# EUROPEAN PATENT APPLICATION

(11) **EP 1 726 645 A1**
(43) Date of publication of application: **29.11.2006**
(21) Application number: 05719369.0
(22) Date of filing: 22.02.2005
(51) Int. Cl.: C12N 15/09, C12N 9/04, C12P 17/12, C12P 17/16

(54) **PROCESS FOR PRODUCING PICOLINIC ACID COMPOUNDS**

(30) Priority: 04.03.2004 JP 2004061238
(71) Applicant: MARINE BIOTECHNOLOGY INSTITUTE CO., LTD., Kamaishi-shi, Iwate 026-0001 (JP)
(72) Inventor: SHINDO, Kazutoshi, Takasaki-shi, Gunma 3701201 (JP); KAGAMI, Osamu, Marine Biotechnology Inst. Co. Ltd., Kamaishi-shi, Iwate 0260001 (JP); MISAWA, Norihiko, Marine Biotechn. Inst. Co. Ltd., Kamaishi-shi, Iwate 0260001 (JP); FURUKAWA, Kensuke, Fukuoka 8114141 (JP)
(74) Representative: Woods, Geoffrey Corlett
(86) International application number: PCT/JP2005/002755
(87) International publication number: WO 2005/085435

(57) **Abstract**

The present invention relates to a method for producing a picolinic acid compound. Specifically, the present invention relates to a method for producing a picolinic acid compound, which comprises reacting an aromatic compound that contains a phenyl group represented by the following formula (I), (II), or (III) with aromatic ring dioxygenase, aromatic ring dihydrodiol dehydrogenase, and aromatic ring diol dioxygenase, and obtaining a picolinic acid compound (I'), (II'), or (III'). 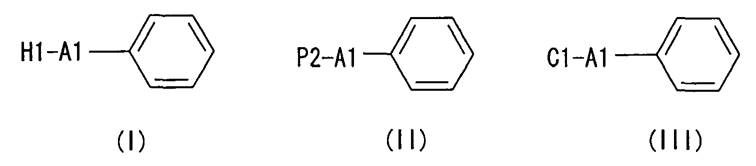 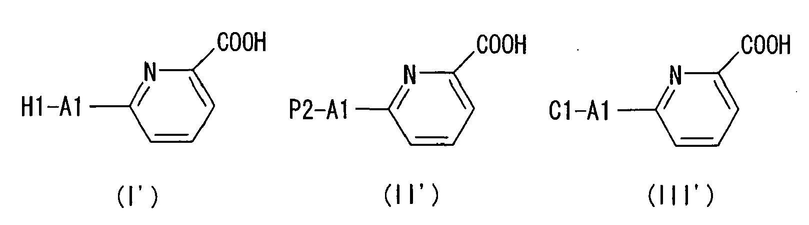 wherein, H1 is an optionally substituted heterocyclic group, A1 is a single bond or an optionally substituted C₁₋₄ alkylene group or alkenylene group, P2 is an optionally substituted phenyl group, and C1 is an optionally substituted cyclic hydrocarbon group (excluding a phenyl group), and where formula II does not represent diphenylacetylene.

## Description

### TECHNICAL FIELD

The technical field to which the present invention belongs is the biocatalytic engineering of low-molecular-weight organic compounds. Specifically, the present invention relates to the production of industrially useful low-molecular-weight organic compounds (industrial raw materials) through bioconversion (also referred to as biotransformation) using recombinant microorganisms such as recombinant *Escherichia coli.* More specifically, the present invention relates to a method for producing organic compounds (picolinic acid compounds) using aromatic compounds that contain various phenyl groups (benzene rings) as raw materials, wherein the phenyl groups (benzene rings) are substituted with picolinic acid groups.

### BACKGROUND ART

*Pseudomonas pseudoalcaligenes* (KF707 strain) is a bacterium that degrades biphenyl and polychlorinated biphenyl (PCB), and it has been isolated in Kitakyushu in Japan. A gene cluster encoding biphenyl catabolic enzymes has been isolated for the first time in the world from the bacteria (Furukawa, K. and Miyazaki, T., "Cloning of gene cluster encoding biphenyl and chlorobiphenyl degradation in Pseudomonas pseudoalcaligenes" J. Bacteriol., Vol. 166, pp. 392-398, 1986). Currently, many studies have been conducted using such biphenyl catabolic enzyme gene cluster derived from such biphenyl-degrading bacteria, because of environmental remediation needs. Many findings concerning the structures or the functions of genes and enzymes have been accumulated (see e.g., Furukawa, K., "Engineering dioxygenases for efficient degradation of environmental pollutants," Curr. Opinion Biotechnol., Vol. 11, pp. 244-249, 2000).

The functions of biphenyl catabolic enzymes, which are exerted in the first 4 reactions, are shown below (Suenaga, H., Goto, M., and Furukawa, K., "Emergence of multifunctional oxygenase activities by random priming recombination" J. Biol. Chem., Vol. 276, pp. 22500-22506, 2001).

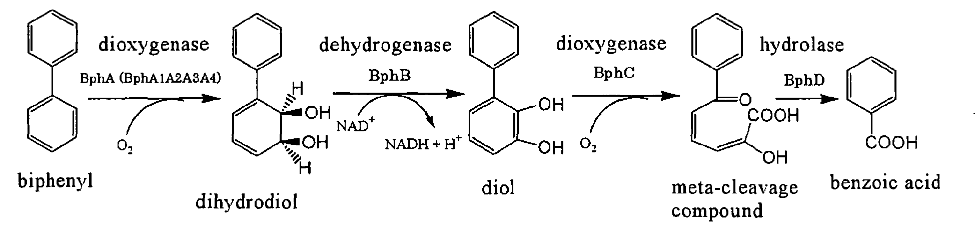

It is thought that when all of the above genes (that is, the bphA (bphA1A2A3A4), bphB, and bphC genes) are introduced into and expressed in *Escherichia coli,* the *Escherichia coli* can metabolize biphenyl to generate a meta-cleavage compound. The ring cleavage product has low added value, because it has almost no applications as a raw material for organic chemical synthesis. The meta-cleavage compound is converted by BphD into benzoic acid.

Successful cases of preparing *cis*-dihydrodiol products from aromatic compounds containing various phenyl groups and the like have already been reported, wherein a biphenyl dioxygenase gene (bphA) derived from the *Pseudomonas pseudoalcaligenes* KF707 strain has been modified as an aromatic ring dioxygenase gene by molecular evolution engineering techniques, the gene has been introduced into and expressed in *Escherichia coli* or actinomycete, and then the *cis*-dihydrodiol products have been prepared using such *E. coli* or actinomycete (see Misawa, N., Shindo, K., Takahashi, H., Suenaga, H., Iguchi, K., Okazaki, H., Harayama, S., and Furukawa, K., "Hydroxylation of various molecules including heterocyclic aromatics using recombinant Escherichia coli cells expressing modified biphenyl dioxygenase genes" Tetrahedron, Vol. 58, pp. 9605-9612, 2002; Shindo, K., Nakamura, R., Chinda, I., Ohnishi, Y. Horinouchi, S., Takahashi, H., Iguchi, K., Harayama, S., Furukawa, K., and Misawa, N., "Hydroxylation of ionized aromatics including carboxylic acid or amine using recombinant Streptomyces lividans cells expressing modified biphenyl dioxygenase genes" Tetrahedron, Vol. 59, pp. 1895-1900, 2003; or JP Patent Publication (Kokai) 2003-269 A). Specifically, DNA shuffling has been performed between DNA encoding a large subunit (BphA1) of biphenyl dioxygenase (isolated from the *Pseudomonas pseudoalcaligenes* KF707 strain, the bacterium that degrades biphenyl and PCB) and DNA encoding a large subunit (BphA1) of biphenyl dioxygenase derived from the LB400 strain of the genus *Burkholderia,* another biphenyl-degrading bacterium, thereby preparing a gene [referred to as the bphA1 (2072) gene] with a broader substrate specificity spectrum. Preparation of a modified biphenyl dioxygenase gene (cluster) has also been reported, comprising the bphA1 (2072) gene and a gene (bphA2A3A4 gene) encoding 3 components other than the large subunit of biphenyl dioxygenase derived from the *Pseudomonas pseudoalcaligenes* KF707 strain. Whether or not aromatic compounds containing various phenyl groups and the like can be converted (can be recognized as substrates) has been examined using transformants of *Escherichia coli* (actinomycetes in some cases) that have had such genes introduced and expressed therein. It has been discovered that bioconversion of a low-molecular-weight organic compound (e.g., 2-phenylquinoline, 2-benzylbenzoxazole, 2-phenylindole, 2-phenylpyridine, 1-phenylpyrazole, 1-benzylimidazole, or 1-benzyl-4-pyrrolidone, containing a heterocycle and a phenyl group within a molecule) that has never been reported so far although the same type of conversion experimentation broadly conducted to date is possible (see Misawa, N., Shindo, K., Takahashi, H., Suenaga, H., Iguchi, K., Okazaki, H., Harayama, S., and Furukawa, K., "Hydroxylation of various molecules including heterocyclic aromatics using recombinant Escherichia coli cells expressing modified biphenyl dioxygenase genes." Tetrahedron, Vol. 58, pp. 9605-9612, 2002; Shindo, K., Nakamura, R., Chinda, I., Ohnishi, Y. Horinouchi, S., Takahashi, H., Iguchi, K., Harayama, S., Furukawa, K., and Misawa, N., "Hydroxylation of ionized aromatics including carboxylic acid or amine using recombinant Streptomyces lividans cells expressing modified biphenyl dioxygenase genes" Tetrahedron, Vol. 59, pp. 1895-1900, 2003). Specifically, it is known that *Escherichia coli* containing modified biphenyl dioxygenase genes [bphA1 (2072) bphA2A3A4; bphA (2072)] that have been subjected to DNA shuffling converts the above low-molecular-weight organic compound, so that it is able to stereoselectively generate an aromatic-*cis*-dihydrodiol product wherein 2 hydroxyl groups and 2 hydrogens have been introduced in a position-specific manner at positions adjacent to each other (when a position in which a substituent is present is designated as 1, positions 2 and 3) within a phenyl group.

Furthermore, the present inventors have discovered that an aromatic-*cis-*dihydrodiol product (synthesized by *Escherichia coli* having the above bphA (2072)) is converted by aromatic ring dihydrodiol dehydrogenase (BphB) derived from the *Pseudomonas pseudoalcaligenes* KF707 strain into a diol product (see JP Patent Application No. 2003-57867 (filed on March 4, 2003)). The present inventors have also discovered that *Escherichia coli* expressing the above bphA (2072) gene and bphB gene can convert flavonoids such as flavone, flavanone, and 6-hydroxyflavone into diol products (see Shindo, K., Kagiyama, Y., Nakamura, R., Hara, A., Ikenaga, H., Furukawa, K., and Misawa, N., "Enzymatic synthesis of novel antioxidant flavonoids by Escherichia coli cells expressing modified metabolic genes involved in biphenyl catabolism" J. Mol. Catalysis B: Enzymatic Vol. 23, pp. 9-16, 2003).

Meanwhile, it is very complicated and difficult to obtain a compound having picolinic acid within a molecule (herein referred to as a "picolinic acid compound") (6-substituent-pyridine-2-carboxylic acid) by chemical synthesis. Almost no methods for producing such picolinic acid compound based on a bioengineering method are known. There have been only 3 reports as follows. The 1^{st} report discloses successful isolation (from soil) of the genus *Pseudomonas* (*Pseudomonas* sp.) that generates 6-phenyl-pyridine-2-carboxylic acid (a type of picolinic acid compound) from biphenyl and a method for producing the 6-phenyl-pyridine-2-carboxylic acid using such microorganism (JP Patent Publication (Kokai) No. 55-000307 A (1980)). The 2^{nd} report discloses that a compound composed of catechols or catechol derivatives such as 3-methylcatechol with one substituent bound thereto is converted into a meta-cleavage product using *Escherichia coli* that expresses a catechol-2,3-dioxygenase gene, the product is then subjected to chemical treatment (that is, agitation in 25% ammonia water or autoclaving), and thus the product can be converted into picolinic acid (Asano, Y., Yamamoto, Y., and Yamada, H., "Catechol 2,3-dioxygenase-catalyzed synthesis of picolinic acids from catechols" Biosci. Biotech. Biochem. Vol. 58, pp. 2054-2056, 1994). The 3^{rd} report discloses that diphenylacetylene is converted into a meta-cleavage product using toluene-degrading bacteria of the genus *Acinetobacter,* the product is then subjected to chemical treatment similar to that of the above report, and then the product is converted into 6-phenylacetylene picolinic acid (Spain, J. C., Nishino, S. F., Witholt, B., Tan, L. -S., and Duetz, W. A., "Production of 6-phenylacetylene picolinic acid from diphenylacetylene by a toluene-degrading Acinetobacter strain" Appl. Environ. Microbiol. Vol. 69, pp. 4037-4042, 2003). The importance of such picolinic acid compounds is expected with regard to pharmaceutical or agricultural chemical intermediates or chemical products with added value. However, as described above, there is currently very little information concerning such production method.

Therefore, the development of a convenient and efficient method for preparing various picolinic acid compounds has been desired.

### DISCLOSURE OF THE INVENTION

An object of the present invention is to provide a convenient and efficient method for producing a picolinic acid compound.

As a result of intensive studies to achieve the above object, the present inventors have discovered that when recombinant *Escherichia coli* cells into which genes encoding aromatic ring (biphenyl) dioxygenase (BphA1A2A3A4: BphA), aromatic ring (biphenyl) dihydrodiol dehydrogenase (BphB), and aromatic ring (biphenyl) diol dioxygenase (BphC) have been introduced and expressed are subjected to mixed culture in a synthetic medium in the presence of a low-molecular-weight organic compound having a phenyl group (benzene ring) within a molecule, not a meta-cleavage compound but a picolinic acid compound (6-substituent-pyridine-2-carboxylic acid) is generated. Thus, the present inventors have completed the present invention. In addition, the present invention does not require any chemical treatment as described in the Background Art.

The present invention encompasses the following inventions.

### (1) A method for producing a picolinic acid compound, which comprises

reacting an aromatic compound that contains a phenyl group represented by the following formula (I), (II), or (III): 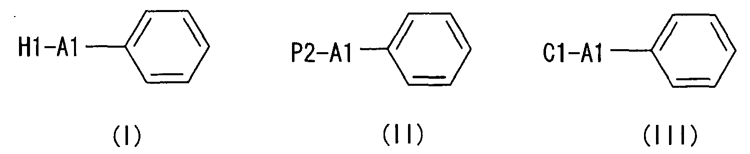 [wherein,
H1 is an optionally substituted heterocyclic group,
A1 is a single bond or an optionally substituted C₁₋₄ alkylene group or alkenylene group,
P2 is an optionally substituted phenyl group, and
C1 is an optionally substituted cyclic hydrocarbon group (excluding a phenyl group), and
where formula II does not represent diphenylacetylene]
with aromatic ring dioxygenase, aromatic ring dihydrodiol dehydrogenase, and aromatic ring diol dioxygenase, and
obtaining a picolinic acid compound (I'), (II'), or (III'): 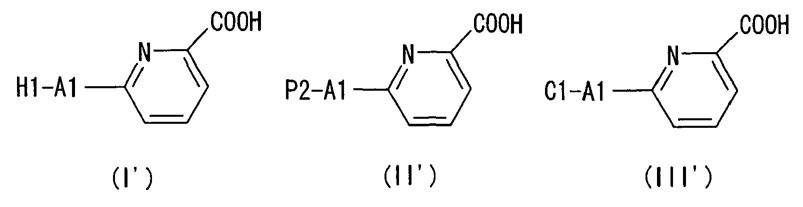 [wherein H1, A1, P2, and C1 are as defined above].

In the above method for producing a picolinic acid compound, aromatic ring dioxygenase, aromatic ring dihydrodiol dehydrogenase, and aromatic ring diol dioxygenase can be those derived from biphenyl-degrading bacteria or can be variants thereof or modified products obtained therefrom by molecular evolution engineering techniques.

### (2) A method for producing a picolinic acid compound, which comprises

culturing a recombinant microorganism into which genes encoding aromatic ring dioxygenase, aromatic ring dihydrodiol dehydrogenase, and aromatic ring diol dioxygenase have been introduced in a medium containing a compound represented by the following formula (I), (II), or (III): 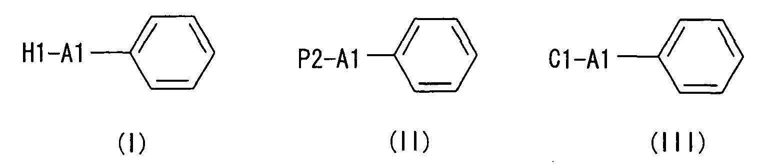 [wherein
H1 is an optionally substituted heterocyclic group,
A1 is a single bond or an optionally substituted C₁₋₄ alkylene group or alkenylene group,
P2 is an optionally substituted phenyl group, and
C1 is an optionally substituted cyclic hydrocarbon group (excluding a phenyl group), and
where formula II does not represent diphenylacetylene] and
obtaining from the culture product or microorganisms a picolinic acid compound (I'), (II'), or (III'): 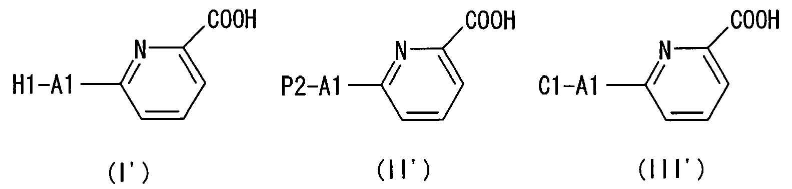 [wherein, H1, A1, P2, and C1 are as defined above].

In the above method for producing a picolinic acid compound, the genes encoding aromatic ring dioxygenase, aromatic ring dihydrodiol dehydrogenase, and aromatic ring diol dioxygenase can be those derived from biphenyl-degrading bacteria or can be variants thereof or modified products obtained therefrom by molecular evolution engineering techniques. Furthermore, an example of a recombinant microorganism is recombinant *Escherichia coli.*

In the above method for producing a picolinic acid compound, examples of the compound represented by the formula (I), (II), or (III) include flavanone, flavone, 6-hydroxyflavanone, 6-hydroxyflavone, 7-hydroxyflavanone, 2-phenylquinoline, 2-phenylbenzoxazole, biphenyl, (trans-)chalcone, 3-phenyl-1-indanone, and 2-phenylnaphthalene.

Moreover, in the above method for producing a picolinic acid compound, the following protein (a) or (b) can be used as a large subunit of aromatic ring dioxygenase:
(a) a protein consisting of the amino acid sequence shown in SEQ ID NO: 2; or
(b) a protein containing a sequence derived from the amino acid sequence shown in SEQ ID NO: 2 by deletion, substitution, or addition of 1 or several amino acids and having functions of the large subunit of aromatic ring dioxygenase;

### (3) A protein (a) or (b) shown below:

(a) a protein containing the amino acid sequence shown in SEQ ID NO: 2; or
(b) a protein containing a sequence derived from the amino acid sequence shown in SEQ ID NO: 2 by deletion, substitution, or addition of 1 or several amino acids and having functions of a large subunit of aromatic ring dioxygenase.

### (4) A gene encoding the following protein (a) or (b):

(a) a protein containing the amino acid sequence shown in SEQ ID NO: 2; or
(b) a protein containing a sequence derived from the amino acid sequence shown in SEQ ID NO: 2 by deletion, substitution, or addition of 1 or several amino acids and having functions of the large subunit of aromatic ring dioxygenase.

### (5) A gene containing DNA consisting of the nucleotide sequence shown in SEQ ID NO: 1.

According to the present invention, a novel method for producing a picolinic acid compound is provided, wherein the picolinic acid compound is a low-molecular-weight organic compound and is produced with difficulty by organic chemical synthesis. In the method according to the present invention, a low-molecular-weight organic compound having a phenyl group (benzene ring) within a molecule as a substrate for enzyme reaction may be used. Hence, raw materials for industrial products having high added value, such as pharmaceutical preparations or agricultural chemicals, can be produced from an abundant variety of inexpensive commercial chemosynthetic substrates. Furthermore, a method for converting a phenyl group (benzene ring) into picolinic acid is provided according to the present invention. The method is useful in terms of provision of a technique for an organic chemical synthesis method.

### Best Mode of Carrying Out the Invention

The present invention is explained in detail as follows. This application claims priority of Japanese patent application No. 2004-61238 filed on March 4, 2004, and encompasses the content described in the description and/or drawings of the above patent application.

The method for producing a picolinic acid compound according to the present invention comprises causing biphenyl catabolic enzymes to act on a low-molecular-weight organic compound having a phenyl group (benzene ring), so as to substitute the phenyl group (benzene ring) with picolinic acid.

Specifically, a method for producing an organic compound (6-substituent-pyridine-2-carboxylic acid) is provided, which comprises causing biphenyl dioxygenase [BphA1A2A3A4: BphA], aromatic ring dihydrodiol dehydrogenase (BphB), and aromatic ring diol dioxygenase (BphC) to act sequentially, so that the phenyl group (benzene ring) moiety is substituted with picolinic acid. Such organic compound having a picolinic acid moiety is useful because it can be used as a raw material for various industrial products, including pharmaceutical preparations and agricultural chemicals.

### 1. Biphenyl catabolic enzymes

In the present invention, the activities of the first 3 enzymes of the biphenyl catabolic pathway are used. Such enzymes are aromatic ring (biphenyl) dioxygenase (also herein referred to as "aromatic ring dioxygenase"), aromatic ring (biphenyl)-cis-dihydrodiol dehydrogenase (desaturase) (also herein referred to as "aromatic ring dihydrodiol dehydrogenase"), and aromatic ring diol (biphenyl diol) dioxygenase (also herein referred to as "aromatic ring diol dioxygenase"). Herein, these enzymes are collectively referred to as biphenyl catabolic enzymes.

Originally reported functions of the 3 above types of enzymes are shown in the following figure (Suenaga, H., Goto, M., and Furukawa, K., "Emergence of multifunctional oxygenase activities by random priming recombination" J. Biol. Chem., Vol. 276, pp. 22500-22506, 2001).

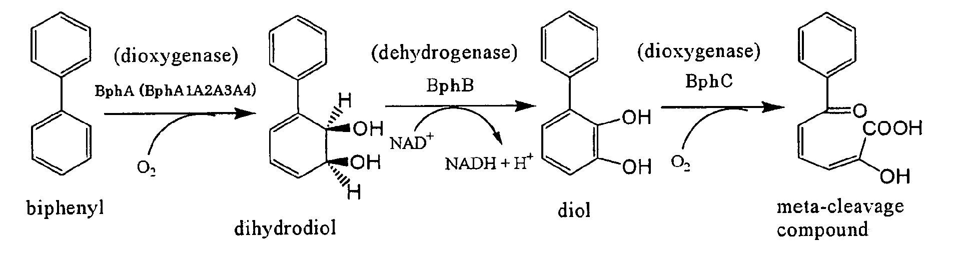

These enzymes can be obtained from microorganisms that naturally produce such enzymes or microorganisms into which genes encoding such enzymes have been introduced. Alternatively, such enzymes can be obtained from microorganisms into which genes encoding variants of such enzymes or modified products obtained therefrom by molecular evolution engineering techniques have been introduced.

### 2. Genes encoding biphenyl catabolic enzymes

The aromatic ring dioxygenase gene consists of bphA1, bphA2, bphA3, and bphA4, the aromatic ring dihydrodiol dehydrogenase gene consists of bphB, and the aromatic ring diol dioxygenase gene consists of bphC. Examples of the aromatic ring dioxygenase gene, the aromatic ring dihydrodiol dehydrogenase gene, and the aromatic ring diol dioxygenase gene herein include not only genes derived from microorganisms having such genes, but also genes encoding variants that are naturally or artificially generated and genes encoding modified products obtained therefrom by molecular evolution engineering techniques, such as DNA shuffling performed between genes (modified aromatic ring dioxygenase gene, modified aromatic ring dihydrodiol dehydrogenase gene, and modified aromatic ring diol dioxygenase gene), for example.

As a bph gene encoding a Bph enzyme that is used in the present invention, such a gene derived from the *Pseudomonas pseudoalcaligenes* KF707 strain can be used, for example. Furthermore, the bphA1 gene [bphA1 (2072)] can be used, for example, where such gene encodes an enzyme with broader substrate specificity as a result of DNA shuffling performed between bphA1 of the *Pseudomonas pseudoalcaligenes* KF707 strain and bphA1 of the LB400 strain of the genus *Burkholderia.* Alternatively, a hybrid bphA1 gene [bphA1 (715-707)] (SEQ ID NO: 1) can be used, where such gene has the bphA1 gene derived from the *Pseudomonas putida* KF715 strain at its central region and has the bphA1 gene of *Pseudomonas pseudoalcaligenes* KF707 strain on both of its ends. Further alternatively, bphA2, bphA3, bphA4, bphB, and bphC genes derived from the *Pseudomonas pseudoalcaligenes* KF707 strain can be used. As a bphA1 gene encoding a large subunit of biphenyl dioxygenase, it is preferable to use bphA1 (2072) or [bphA1 (715-707)] that is a modified gene of a large subunit of biphenyl dioxygenase, where such gene has better conversion efficiency than that of such a gene derived from the *Pseudomonas pseudoalcaligenes* KF707 strain.

The amino acid sequence of the former modified large subunit [BphA1(2072)] of biphenyl dioxygenase is shown in DDBJ/Genbank accession No. AB085748. Moreover, the amino acid sequences of a small subunit (BphA2), ferredoxin (BphA3), and ferredoxin reductase (BphA4) of the KF707 strain are shown in DDBJ/Genbank accession No. M83673. The amino acid sequence of BphB is shown in DDBJ/Genbank accession No. M83673. In addition, the amino acid sequence of aromatic ring diol dioxygenase (BphC) that catalyzes the next reaction is shown in DDBJ/Genbank accession No. M83673.

An example of the bphA1 (715-707) gene (SEQ ID NO: 1) that can be used in the present invention is the hybrid bphA1 gene having the bphA1 sequence isolated from the *Pseudomonas putida* KF715 strain ( Hayase, N., Taira, K., and Furukawa, K., "Pseudomonas putida KF715 bphABCD operon encoding biphenyl and polychlorinated biphenyl degradation: Cloning, analysis, and expression in soil bacteria," J. Bacteriol., 172, 1160-1164, 1990) at its central region and having the bphA1 sequence derived from the *Pseudomonas pseudoalcaligenes* KF707 strain on both of its ends. The bphA1 (715-707) gene has been constructed after isolation of the bphA1 sequence from the *Pseudomonas putida* KF715 strain with the use of a cassette PCR technique (JP Patent Publication (Kokai) No. 2000-125871 A). The bphA1 (715-707) gene has good conversion efficiency for enzyme reactions. The nucleotide sequence of the gene is shown in SEQ ID NO: 1 and the amino acid sequence of the gene is shown in SEQ ID NO: 2.

As long as a protein containing the amino acid sequence shown in SEQ ID NO: 2 has functions of a large subunit of aromatic ring dioxygenase, mutation such as deletion, substitution, or addition of a plurality of and preferably 1 or 2 amino acids may be incorporated into the amino acid sequence. The functions of a large subunit of aromatic ring dioxygenase indicate possession of the functions of a major constitutive protein in an aromatic ring dioxygenase enzyme, such functions being responsible for substrate recognition and oxygenation reaction. Such functions can be confirmed by conducting a substrate bioconversion experiment wherein co-expression within *Escherichia coli* with a small subunit of aromatic ring dioxygenase, ferredoxin, and a ferredoxin reductase gene is performed.

An example of a protein having functions of the large subunit of an aromatic ring dioxygenase of the present invention is a protein having a sequence prepared by deletion of 1 to 2 amino acids from the amino acid sequence shown in SEQ ID NO: 2, addition of 1 to 2 amino acids to the amino acid sequence shown in SEQ ID NO: 2, or substitution of 1 to 2 amino acids in the amino acid sequence shown in SEQ ID NO: 2 with other amino acids.

In addition, the above large subunit of aromatic ring dioxygenase is useful not only in production of a picolinic acid compound that is described later, but also for production of dihydrodiol products or diol products and, furthermore, for the degradation reaction of an aromatic compound (e.g., biphenyl or PCB).

JM109 (pBPA715-707BC) contains an aromatic ring dioxygenase gene [bphA1 (715-707) A2A3A4] (wherein the bphA1 (715-707) gene has been ligated upstream of the bphA2A3A4 gene derived from the *Pseudomonas pseudoalcaligenes* KF707 strain), and the aromatic ring dihydrodiol dehydrogenase gene (bphB) and aromatic ring diol dioxygenase (bphC) derived from the *Pseudomonas pseudoalcaligenes* KF707 strain incorporated therein. JM109 (pBPA715-707BC) was internationally deposited under the Budapest Treaty on February 2, 2004 (original deposition) under accession No. FERM BP-10210 with the International Patent Organism Depositary (Tsukuba Central 6, 1-1-1 Higashi, Tsukuba, Ibaraki, Japan), the National Institute of Advanced Industrial Science and Technology.

Furthermore, the above-described genes encoding biphenyl catabolic enzymes may be variants wherein mutation has been introduced by site-directed mutagenesis or the like and which have the above functions or activity. In addition, to introduce mutation into a gene, a known technique such as a Kunkel method or a Gapped duplex method, or a method according thereto, can be employed. For example, mutation is introduced using a kit for introducing mutation (e.g., Mutan-K (produced by TAKARA) or Mutan-G (produced by TAKARA)) using site-directed mutagenesis. Moreover, mutation can be introduced into a gene or a chimeric gene can also be constructed by techniques such as error-prone PCR or DNA shuffling. Error-prone PCR and DNA shuffling techniques are known in the technical field. For example, regarding error-prone PCR, see Chen, K., and Arnold, F. H., 1993, Proc. Natl. Acad. Sci. U. S. A., 90: 5618-5622. Regarding molecular evolution engineering techniques such as DNA shuffling and cassette PCR, see Kurtzman, A. L., Govindarajan, S., Vahle, K., Jones, J. T., Heinrichs, V., Patten P. A., "Advances in directed protein evolution by recursive genetic recombination: applications to therapeutic proteins" Curr. Opinion Biotechnol., 12, 361-370, 2001, and Okuta, A., Ohnishi, A., and Harayama, S., "PCR isolation of catechol 2,3-dioxygenase gene fragments from environmental samples and their assembly into functional genes" Gene, 212, 221-228, 1998, for example.

Furthermore, the genes of a gene cluster encoding the above biphenyl catabolic enzymes are generally present adjacent to each other or very close to each other within a genomic DNA or plasmid DNA. Hence, if a clone containing the sequence of any one of these genes, such as a cosmid clone, is isolated by a colony hybridization method or the like, the other genes can often be simply isolated together.

However, a number of genes encoding proteins having similar enzymatic activities have been reported and are known by persons skilled in the art. The present invention is not limited to those using enzymes that are produced by recombinant microorganisms having these gene sequences.

### 3. Microorganisms producing biphenyl catabolic enzymes

Examples of microorganisms that naturally produce biphenyl catabolic enzymes include biphenyl-degrading bacteria (that degrade biphenyl, PCB (polychlorinated biphenyl), and the like) of the genus *Pseudomonas,* the genus *Comamonas,* the genus *Burkholderia,* the genus *Sphingomonas,* the genus *Rhodoccus,* the genus *Ralstonia,* and the like. Examples of bacteria belonging to these genera include *Pseudomonas pseudoalcaligenes, Comamonas testosteroni, Burkholderia* LB400, *Sphingomonas aromaticivorans, Rodococcus globerulus,* and *Ralstonia oxalatica.* These bacteria can be obtained from culture collections such as ATCC and DSMZ. However, microorganisms that can be used in the present invention are not limited to the above bacteria. Any microorganism can be used, as long as it has at least one gene that encodes an enzyme having aromatic ring dioxygenase activity, aromatic ring dihydrodiol dehydrogenase activity, or aromatic ring diol dioxygenase activity.

As an example of a microorganism having an aromatic ring dioxygenase gene, an aromatic ring dihydrodiol dehydrogenase gene, or an aromatic ring diol dioxygenase gene, after analysis of the *Pseudomonas pseudoalcaligenes* KF707 strain (Furukawa, K. and Miyazaki, T., "Cloning of gene cluster encoding biphenyl and chlorobiphenyl degradation in Pseudomonas pseudoalcaligenes" J. Bacteriol., Vol. 166, pp. 392-398, 1986) and the LB400 strain of the genus *Burkholderia* (close to the genus *Pseudomonas*) (Erickson, B. D., Mondello, F. J., "Nucleotide sequencing and transcriptional mapping of the genes encoding biphenyl dioxygenase, a multicomponent polychlorinated-biphenyl-degrading enzyme in Pseudomonas strain LB400" J. Bacteriol., 174, 2903-2912, 1992) biphenyl catabolic enzyme genes have been isolated. JP Patent Publication (Kokai) 2003-000269 A discloses *Escherichia coli* JM109 (pKF6622) having the aromatic ring dioxygenase gene (bphA1A2A3A4) derived from the *Pseudomonas pseudoalcaligenes* KF707 strain incorporated therein. Furthermore, JP Patent Publication (Kokai) No. 2003-000269 A discloses *Escherichia coli* JM109 (pKF2072) having an aromatic ring dioxygenase gene [bphA1(2072)A2A3A4] incorporated therein, wherein the gene contains a bphA1 (2072) gene that is prepared through DNA shuffling between bphA1 derived from the *Pseudomonas pseudoalcaligenes* KF707 strain and bphA1 derived from the LB 400 strain of the genus *Burkholderia,* so as to cause molecular evolution.

In the present invention, recombinant *Escherichia coli* can be used, for example, wherein the genes explained in the above section "2. Genes encoding biphenyl catabolic enzymes" have been introduced and expressed. Such genes are modified aromatic ring (modified biphenyl) dioxygenase gene [bphA1(2072)A2A3A4] (also abbreviated as bphA(2072)) or [bphA1(715-707)A2A3A4] (may also be abbreviated as bphA(715-707)); the aromatic ring (biphenyl) dihydrodiol dehydrogenase (bphB) gene; and the aromatic ring (biphenyl) diol dioxygenase (bphC) gene. Furthermore, enzymes that are produced by such recombinant *Escherichia coli* can be used.

A method for introducing and expressing a foreign gene in *Escherichia coli* can be performed according to a standard method (e.g., Sambrook, J., Russell, D. W., "Molecular Cloning; A Laboratory Manual," Third edition, Cold Spring Harbor Laboratory Press, 2001). For example, with the use of pBluescript II SK as an *Escherichia coli* vector, a lac promoter can be used for expressing the bphA(2072)BC gene or the bphA (715-707) BC gene.

In addition, a microorganism to be used as a host is not limited to *Escherichia coli.* For example, the above gene can be introduced into and expressed by bacteria of the genus *Streptomyces,* which are actinomycetes. Such transformed recombinant microorganisms can also be used (Chun, H. K., Ohnishi, Y., Shindo, K., Misawa, N., Furukawa, K., Horinouchi, S., "Biotransformation of flavone and flavanone by Streptomyce lividans cells carrying shuffled biphenyl dioxygenase gene". J. Mol. Catalysis B: Enzymatic, 21, 113-121, 2003).

### 4. Isolation and purification of biphenyl catabolic enzymes

In the present invention, the relevant enzymes are isolated and purified from microorganisms producing biphenyl catabolic enzymes. The enzymes can then be used for the conversion reaction described later.

Such biphenyl catabolic enzymes can be obtained by culturing the above microorganisms possessing genes that encode such enzymes and then recovering the enzymes from the culture products. "Culture products" mean any of culture supernatants, cultured cells, cultured microorganisms, and disrupted cells or disrupted microorganisms. In the present invention, a method for culturing microorganisms in a medium is performed according to a general method that is employed for culturing microorganisms.

As a medium for culturing microorganisms such as bacteria, either a natural or a synthetic medium may be used, as long as it contains carbon sources, nitrogen sources, inorganic salts, and the like, assimilable by microorganisms, and enables efficient culture of microorganisms.

Examples of carbon sources that are used herein include carbohydrates (e.g., glucose, fructose, sucrose, and starch), organic acids (e.g., acetic acid and propionic acid), alcohols (e.g., ethanol and propanol), and aromatic hydrocarbons (e.g., dibenzofuran, gentisic acid, and salicylic acid). Examples of nitrogen sources that are used herein include ammonia, inorganic or organic acid salts of ammonium (e.g., ammonium chloride, ammonium sulfate, ammonium acetate, and ammonium phosphate), peptone, meat extract, corn steep liquor, and other nitrogen-containing compounds. Examples of inorganic substances that are used herein include primary potassium phosphate, secondary potassium phosphate, magnesium phosphate, magnesium sulfate, sodium chloride, ferrous sulfate, manganese sulfate, copper sulfate, and calcium carbonate.

Culture is generally carried out under aerobic conditions such as shake culture or aeration and agitation culture at approximately 20°C to 40°C. During a culture period, pH is maintained between approximately 5 and 9. pH is adjusted using inorganic or organic acid, an alkaline solution, or the like. During culture, antibiotics such as ampicillin or tetracycline may be added to a medium, if necessary.

After culture, when target biphenyl catabolic enzyme proteins are produced within microorganisms or within cells, the proteins are extracted by disrupting such microorganisms or cells. Furthermore, when target proteins are produced outside the microorganisms or cells, the culture solution is used intact or the microorganisms or cells are removed by centrifugation or the like. Subsequently, target proteins can be isolated and purified from the above culture products using one of or an appropriate combination of the general biochemical methods that are employed for protein isolation and purification, including ammonium sulfate precipitation, gel chromatography, ion exchange chromatography, affinity chromatography, and the like.

Whether or not target proteins can be obtained can be confirmed by SDS-polyacrylamide gel electrophoresis or the like. Moreover, various tests can be conducted to examine the physicochemical properties or functions of target proteins. Examples of test items include X-ray crystal analysis, CD spectrum analysis, and NMR analysis.

### 5. Conversion reaction to picolinic acid compounds

The method for producing a picolinic acid compound according to the present invention comprises, as described above, with the use of aromatic ring dioxygenase, aromatic ring dihydrodiol dehydrogenase, and aromatic ring diol dioxygenase that are produced by a microorganism having an aromatic ring dioxygenase gene, an aromatic ring dihydrodiol dehydrogenase gene, and an aromatic ring diol dioxygenase gene, or modified genes thereof,
reacting an aromatic compound that contains a phenyl group represented by the following formula (I), (II), or (III): 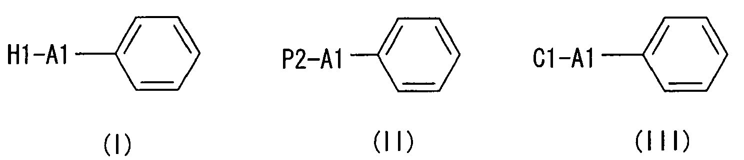 [wherein,
H1 is an optionally substituted heterocyclic group,
A1 is a single bond or an optionally substituted C₁₋₄ alkylene group or alkenylene group,
P2 is an optionally substituted phenyl group, and
C1 is an optionally substituted cyclic hydrocarbon group (excluding a phenyl group), and
where formula II does not represent diphenylacetylene]
with aromatic ring dioxygenase, aromatic ring dihydrodiol dehydrogenase, aromatic ring diol dioxygenase, and meta-cleavage compound hydrolase, and
obtaining a picolinic acid compound (I'), (II'), or (III'): 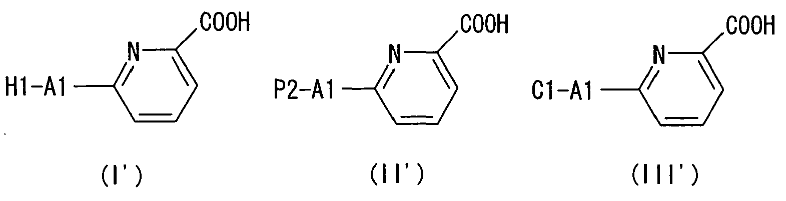 [wherein H1, A1, P2, and C1 are as defined above].

Here, "picolinic acid compound" means a compound that can be generally represented by 6-substituent-pyridine-2-carboxylic acid and has a structure wherein a heterocyclic group, a phenyl group, or a cyclic hydrocarbon group is bound directly to or via an alkylene group or an alkenylene group to position 6 of picolinic acid (pyridine-2-carboxylic acid).

"Heterocyclic group" herein means a monocyclic or bicyclic group comprising one or more heteroatoms selected from the group consisting of a nitrogen atom, an oxygen atom, and a sulfur atom as a cyclic atom, and it may be optionally substituted with a substituent. Examples of "heterocyclic group" include a 5- to 7-membered saturated or unsaturated monocyclic heterocyclic group that may be optionally substituted with a C₁₋₄ alkyl group and a 9- to 11-membered saturated or unsaturated bicyclic heterocyclic group that may be optionally substituted with a C₁₋₄ alkyl group. Specific examples of a heterocycle composing "heterocyclic group" include quinoline, indole, indanone, benzothiazole, benzoxazole, pyridine, 3-methylpyridine, pyrimidine, pyrrole, pyrazole, 3-methylpyrazole, imidazole, isothiazole, benzofuran, thiophene, chromone (4H-chromene-4-one), chroman-4-one, 6-hydroxy-chroman-4-one, and phthalimide.

An alkylene group is -(CH₂)n- (wherein n is an integer between 1 and 4). Examples of such alkylene group include a methylene group, an ethylene group, a trimethylene group, a tetramethylene group, an ethylidene group, an isopropylidene group, and a propylene group.

Examples of an alkenylene group include a vinylene group, a 1-propenylene group, a 1-butenylene group, and a 2-butenylene group.

The above heterocyclic group, alkylene group, or alkenylene group may contain a structure such as -(C=O)- or -O- within the group. Furthermore, the above heterocyclic group, alkylene group, or alkenylene group may have a substituent. Examples of such substituent include a C₁₋₄ alkyl group, an amino group, a hydroxyl group, and a cyano group.

Moreover, examples of a cyclic hydrocarbon group include a C₅₋₁₄ saturated or unsaturated cyclic hydrocarbon group. Specific examples of such cyclic hydrocarbon group include aromatic compounds such as a 1-indanone group, a 1-naphthyl group, a 2-naphthyl group, an anthranil group, and a phenanthrenyl group.

Examples of a low-molecular-weight organic compound that can be used as a substrate for conversion reaction and has a phenyl group (benzene ring) within a molecule not only include a compound having a biphenyl bond between a heterocycle and a benzene ring (e.g., flavanone, flavone, 6-hydroxyflavanone, 6-hydroxyflavone, 7-hydroxyflavanone, 2-phenylquinoline, and 2-phenylbenzoxazole) but also include a compound having a heterocycle and a benzene ring bound via a methylene group (e.g., 2-benzyl pyridine), a compound having a biphenyl bond between a cyclic hydrocarbon group and a phenyl ring (e.g., biphenyl, 3-phenyl-1-indanone, 1-phenylnaphthalene, and 2-phenylnaphthalene), and a compound having a C₃-substituted alkenylene group between an aromatic ring and a phenyl group (e.g., (trans-)chalcone).

Examples of substrate compounds that are suitably used in the present invention include the following compounds:
Specific examples of compounds represented by formula (I): 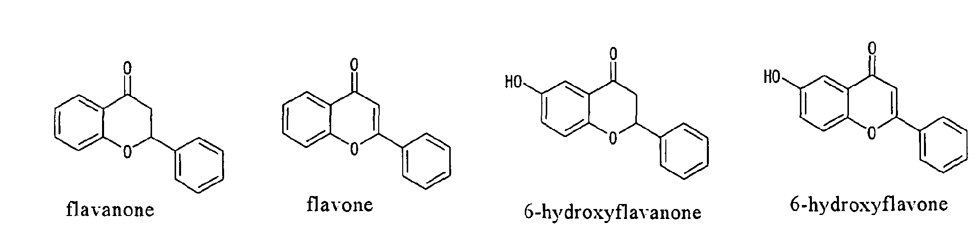 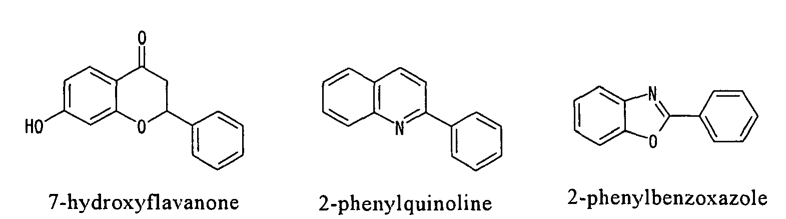
Specific examples of compounds represented by formula (II): 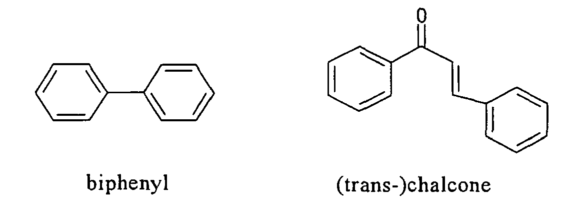
Specific examples of compounds represented by formula (III):

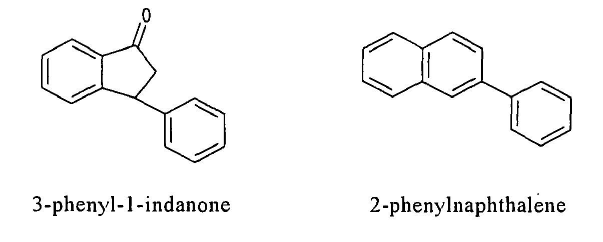

Through reaction of the compounds represented by the above formulae (I) to (III) with aromatic ring dioxygenase, aromatic ring dihydrodiol dehydrogenase, and aromatic ring diol dioxygenase or through culture of microorganisms producing these enzymes with the compounds, phenyl groups (benzene rings) can be substituted with picolinic acid as shown in the following formulae (I') to (III'). 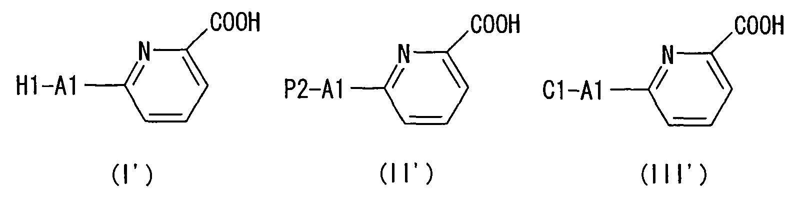 [wherein H1, A1, P2, and C1 are as defined above].

Specifically, according to the method of the present invention, corresponding picolinic acid compounds [compound 1 (from flavanone), compound 2 (from flavone), compound 3 (from 6-hydroxyflavanone), compound 4 (from 6-hydroxyflavone) compound 5 (from 7-hydroxyflavanone), compound 6 (from 2-phenylquinoline), and compound 7 (from 2-phenylbenzoxazole)] as shown below, for example, can be obtained from 7 compounds that are shown as specific examples of formula (I). These compounds are all novel substances.

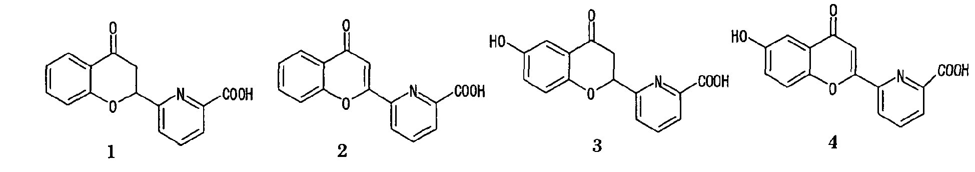

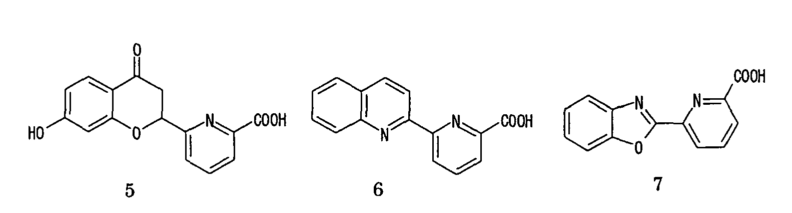

Furthermore, corresponding picolinic acid compounds [compound 8 (from biphenyl) and compound 9 (from (trans-)chalcone)] as shown below can be obtained from 2 compounds that are shown as specific examples of formula (II). Compound 9 is a novel substance.

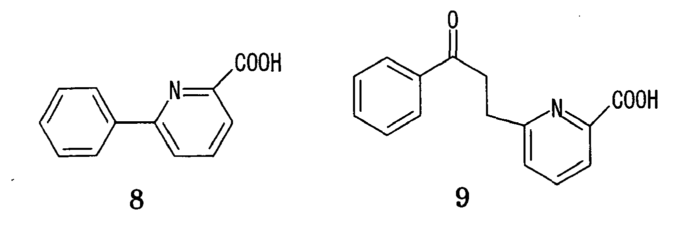

In the above conversion product 9, an alkenylene group (one double bond between carbons) of the original substrate ((trans-)chalcone) was changed to a saturated alkylene group.

Moreover, corresponding picolinic acid compounds [compound 10 (from 3-phenyl-1-indanone) and compound 11 (from 2-phenylnaphthalene)] shown below can be obtained from 2 compounds shown as specific examples of formula (III). These 2 compounds are novel substances.

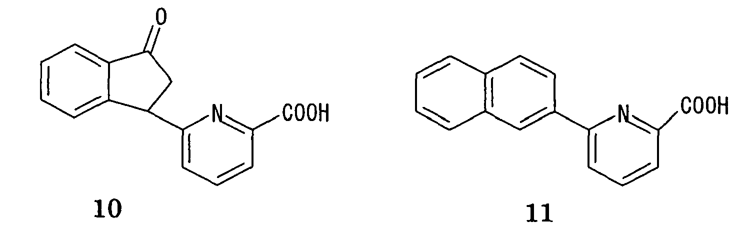

A method that comprises reacting the compounds represented by the above formulae (I) to (III) with aromatic ring dioxygenase, aromatic ring dihydrodiol dehydrogenase, and aromatic ring diol dioxygenase (alternatively, disrupted microorganisms containing these enzymes, culture solutions of microorganisms containing these enzymes, crude enzymes, purified enzymes, or the like) or that comprises culturing the compounds with microorganisms that produce the above 3 enzymes can be conducted according to a standard method in a manner similar to a general enzyme reaction method or culture method.

For example, such a method for culturing microorganisms that produce aromatic ring dioxygenase, aromatic ring dihydrodiol dehydrogenase, and aromatic ring diol dioxygenase with the compounds represented by the above formulae (I) to (III) can be conducted as described below.

In general, a medium for culturing microorganisms may be any medium in which the microorganisms can grow. Specific examples of such medium include an LB medium, an M9 medium, a KB medium, a YM medium, a KY medium, and an F101 medium.

As a carbon source, any carbon compound can be used, as long as it is assimilable by microorganisms and enables the growth of microorganisms. As nitrogen sources, inorganic nitrogen sources such as ammonium sulfate, ammonium chloride, and ammonium nitrate and organic nitrogen sources such as yeast extract, peptone, and meat extract can be used. In addition to these sources, if necessary, inorganic salts, metal salt, vitamin, and the like can also be added. However, since it is predicted that a nitrogen source for picolinic acid may be ammonia, the use of inorganic nitrogen sources such as ammonium sulfate, ammonium chloride, and ammonium nitrate as major nitrogen sources is desired.

In general, culture is performed at a temperature between 20°C and 40°C and more preferably between 25°C and 35°C, and with preferable pH between 5 and 9. Furthermore, shake culture or rotational culture may be performed appropriately.

After the completion of culture, the culture solution is treated with a centrifuge, the supernatant is recovered, and then the supernatant solution is extracted using an organic solvent such as ethyl acetate. Subsequently, the extracted solution is treated by column chromatography or the like, so that a target picolinic acid compound can be obtained.

### EXAMPLES

The present invention will be further described specifically by referring to examples. However, the present invention is not limited by these examples.

### [Example 1] Construction of plasmid pSHF1072 for simultaneous expression of modified aromatic ring dioxygenase [bphA(2072)]/aromatic ring dihydrodiol dehydrogenase/aromatic ring diol oxygenase

A gene encoding a large subunit of aromatic ring (biphenyl) dioxygenase derived from the LB400 strain of the genus *Burkholderia* (bphA1) (GenBank accession No. M86348) and a DNA encoding a large subunit of aromatic ring (biphenyl) dioxygenase derived from the *Pseudomonas pseudoalcaligenes* KF707 strain (bphA1) (GenBank accession No. M83673) were isolated by PCR using bphA1 primers comprising consensus flanking sequences. The nucleotide sequences of the bphA1 primers are as follows:
Forward primer 1: 5'-CCGAATTCAAGGAGACGTTGAATCATGAGCTCAGC-3' (SEQ ID NO: 3)
Reverse primer 1: 5'-TTGAATTCTTCCGGTTGACAGATCT-3' (SEQ ID NO: 4)

In addition, the forward primer 1 contains a *Sac* I site and the reverse primer 1 contains a *Bgl* II site. At both ends of the forward and the reverse primers, *Eco*R I sites are added (all of these sites are underlined). PCR was performed for 25 cycles, each consisting of 94°C for 1 minute, 52°C for 1.5 minutes, and 72°C for 1 minute.

The above 2 types of isolated bphA1 were mixed and then the resultant was subjected to degradation treatment using 0.15 units of DnaseI (TAKARA SHUZO) at 15°C for 6 minutes. DNA fragments (10-50 bp) were recovered from agarose gel and then mixed. Self-priming PCR and PCR using the bphA1 primers were performed. Hence, PCR products containing various chimeric bphA1 products wherein amino acid sequences had been randomly replaced (DNA shuffling) were obtained. PCR was performed under conditions that were the same as the above conditions. The PCR products containing various chimeric bphA1 products were subjected to double digestion with *Sac* I/*Bgl* II and then purified from agarose gel.

In the case of *Escherichia coli* having an expression plasmid pJHF18 (Hirose, J., Suyama, A., Hayashida, S., Furukawa, K., Gene, 138, 27-33, 1994) that contains a bphA1A2A3A4-bphB-bphC gene cluster (GenBank accession No. M83673) from the *Pseudomonas pseudoalcaligenes* KF707 strain, the relevant reaction proceeds to meta-cleavage. Hence, when biphenyl is used as a substrate, it is thought that 2-hydroxy-6-oxo-6-phenylhexa-2,4-dienoic acid is generated as a meta-cleavage product. In general, such meta-cleavage product displays yellow, so that it can be monitored at 434 nm. In the plasmid pJHF18, an *Mlu* I site is present at one position within bphA1. Hence, digestion was performed with *Mlu* I, cohesive ends were blunt-ended, and then ligation was performed again, thereby preparing a plasmid pJHF18Δ *Mlu* I wherein bphA1 alone had been disrupted (T. Kumamaru, H. Suenaga, M. Mitsuoka, T. Watanabe, and K. Furukawa, Nature Biotechnology, 16, 663-666, 1998).

Next, pJHF18Δ *Mlu* I was subjected to double digestion with *Sac* I/*Bgl* II, so as to remove 1.39-kb fragment containing the ΔbphA1 gene alone. Instead, PCR products (after double digestion with *Sac* I/*Bgl* II) containing various chimeric products prepared above were inserted, thereby obtaining a series of plasmids (pSHF1000 series) containing various modified aromatic ring dioxygenase genes (modified bphA1::bphA2A3A4 genes) and a bphBbphC gene.

Biphenyl vapor was applied to *Escherichia coli* XL1-Blue having a series of plasmids. Colonies capable of displaying yellow as a result of meta-cleavage were selected and then used for the following experiments. The presence of colonies capable of displaying yellow as a result of meta-cleavage means that the modified bphA1 gene obtained by DNA shuffling can function normally.

Several *Escherichia coli* transformants successfully displayed yellow after application of biphenyl vapor (where plasmids contained in such *Escherichia coli* were designated pSHF1072). One of such *E. coli* transformants was efficient in degrading biphenyl through meta-cleavage, at a level nearly twice as high as that of a transformant having the bphA1 genes of the parents (KF707 and LB400). Furthermore, this transformant (containing pSHF1072 plasmid) having such high degradation efficiency could degrade benzene and toluene through meta-cleavage, whereas the transformant having the bphA1 genes of the parents (KF707 and LB400) was unable to degrade benzene and toluene. However, the degradation efficiency of this transformant was about one third of that of a transformant having a corresponding todC1 gene of *Pseudomonas putida* F1.

A bphA1 gene (derived from the above plasmid pSHF1072) subjected to shuffling is referred to as a bphA1 (2072) gene. The nucleotide sequence of the bphA1 gene is registered with GenBank under accession No. AB085748. Moreover, the nucleotide sequence of bphA2A3A4 is registered with GenBank under accession No. M83673 as described above.

In addition, a plasmid pKF2072 expressing a bphA1(2072)bphA2A3A4 gene has already been prepared from pSHF1072 by deleting bphBC alone. Results of various bioconversion experiments using the *Escherichia coli* JM109 strain into which the plasmid had been introduced are disclosed in the above-mentioned Misawa, N., Shindo, K., Takahashi, H., Suenaga, H., Iguchi, K., Okazaki, H., Harayama, S., and Furukawa, K., "Hydroxylation of various molecules including heterocyclic aromatics using recombinant Escherichia coli cells expressing modified biphenyl dioxygenase genes." Tetrahedron, Vol. 58, pp. 9605-9612, 2002, and JP Patent Publication (Kokai) 2003-000269A.

It is known that *Escherichia coli* (pKF2072) converts heteroaromatic compounds containing various phenyl groups and the like, so as to be able to stereoselectively generate aromatic-*cis*-dihydrodiol products wherein 2 hydroxyl groups and 2 hydrogens have been introduced in a position-specific manner at positions adjacent to each other within a phenyl group or a heteroaromatic group. Of these, a representative example of such reaction specificity is a stereo-specific reaction whereby heteroaromatic group-*cis*-2,3-dihydrobenezenediol is synthesized as a product using an aromatic ring compound (having a single bond (biphenyl bond) between a heteroaromatic group and a phenyl group) as a substrate.

In addition, JP Patent Publication (Kokai) 2003-000269 A discloses a specific method for preparing *Escherichia coli* JM109 (pKF2072) into which the above modified aromatic ring dioxygenase genes have been incorporated.

### [Example 2] Construction of plasmid pBPA715-707BC for simultaneous expression of modified aromatic ring dioxygenase [bphA(715-707)]/aromatic ring dihydrodiol dehydrogenase/aromatic ring diol oxygenase

The structures of large subunits (BphA1) of biphenyl dioxygenase of various biphenyl-degrading bacteria were compared. Thus, two highly conserved amino acid sequences were found to be present among them. Specifically, these were an amino acid sequence (conserved region 1) represented by Asp-Lys-Ser-Ile-Lys-Val-Phe-Leu-Asn-Gln-Cys-Arg (SEQ ID NO: 5) corresponding to 90^{th} to 101^{st} amino acids of the amino acid sequence of BphA1 derived from the *Pseudomonas pseudoalcaligenes* KF707 strain and an amino acid sequence (conserved region 2) represented by Asp-Asp-Gly-Glu-Asn-Trp-Val-Glu-Ile-Gln-Lys-Gly (SEQ ID NO: 6) corresponding to 386^{th} to 397^{th} amino acids of the same.

Biphenyl-degrading bacteria, the *Pseudomonas graminis* KF701 strain, the KF702 strain of the genus *Pseudomonas,* the *Comamonas testosteroni* KF704 strain, the *Sphingomonas yanoikuyae* KF706 strain, the *Ralstonia paucula* KF709 strain, the *Comamonas testosteroni* KF712 strain, the *Psudomonas putida* KF715 strain, and the *Psudomonas putida* KF751 strain, were each cultured overnight in 2 ml of an LB medium at 37°C. Each genomic DNA was extracted according to a standard method. PCR reaction was performed using these genomic DNAs as templates and primers encoding the amino acid sequences of the above two BphA1 conserved regions. An approximately 0.9-kb gene (hereinafter referred to as the A1 cassette region) corresponding to a region flanked by conserved region 1 and conserved region 2 of each gene bphA1 of the large subunit of biphenyl dioxygenase was amplified. Primers used herein were synthesized in the form of mixtures. Such mixtures were prepared through combination of codon sequences with high frequencies (selected from among a plurality of codons encoding the above amino acid sequences) in a plurality of biphenyl-degrading bacteria. The nucleotide sequences of such primers are as shown below:
Forward primer 2: 5'-GACAAGAGCATCAAGGTGTTCCT(A,C,G,T)AACCAGTG (C,T)CG(A,C,G,T)CA-3' (SEQ ID NO: 7)
Reverse primer 2: 5'-CCCCTTCTGGATCTCCACCCAGTT(C,T)TC(A,C,G,T)CC(A,G)TCGTC-3' (SEQ ID NO: 8)

In the sequences, a plurality of the sequences having nucleotides shown in parentheses are mixed. PCR was performed for 30 cycles, each consisting of 94°C for 1 minute, 61°C for 1 minute, and 72°C for 1 minute.

Next, genes encoding an external portion of conserved region 1 (an amino acid sequence ranging from the N-terminus to 95^{th} amino acid) and an external portion of conserved region 2 (an amino acid sequence ranging from 393^{rd} amino acid to the C-terminus), respectively, were amplified by PCR using the bphA1 gene derived from the *Pseudomonas pseudoalcaligenes* KF707 strain as a template. Forward primer 1 (see Example 1) and reverse primer 3 were used for amplification of the partial gene (the amino acid sequence ranging from the N-terminus to 95^{th} amino acid and hereinafter referred to as the 5' arm). Forward primer 3 and reverse primer 1 (see Example 1) were used for amplification of the partial gene (the amino acid sequence ranging from 393^{rd} amino acid to the C-terminus and hereinafter referred to as the 3' arm). Reverse primer 3 and forward primer 3 are as shown below:
Reverse primer 3: 5'- AACACCTTGATGCTCTTGTC -3' (SEQ ID NO: 9)
Forward primer 3: 5'- GGGTGGAGATCCAGAAGGGG -3' (SEQ ID NO: 10)

PCR was performed for 30 cycles, each consisting of 94°C for 1 minute, 61°C for 1 minute, and 72°C for 1 minute.

Next, for each A1 cassette region, the 5'-ann and the 3'-arm were mixed and then PCR reaction was performed by adding forward primer 1 and reverse primer 1. PCR was performed for 25 cycles, each consisting of 94°C for 1 minute, 61°C for 1 minute, and 72°C for 2 minutes. In this PCR reaction, gene fragments were each annealed via overlapping portions. Thus, approximately 1.5-kb genes were each amplified, corresponding to the full-length bphA1 gene. Each amplified gene had a structure of [5' arm-each A1 cassette region-3' arm] and was a hybrid bphA1 gene wherein the portion flanked by conserved region 1 and conserved region 2 of the bphA1 gene derived from the *Pseudomonas pseudoalcaligenes* KF707 strain had been replaced by the bphA1 gene derived from each KF strain. Each of these hybrid bphA1 genes was replaced by the bphA1 gene of the expression plasmid pJHF18 containing the bphA1A2A3A4-bphB-bphC gene cluster of the *Pseudomonas pseudoalcaligenes* KF707 strain. Therefore, various expression plasmids (pBPA7xx-707BC series) containing various modified aromatic ring dioxygenase genes (hybrid bphA1::bphA2A3A4 genes) and the bphBbphC gene were obtained.

Biphenyl vapor was applied to *Escherichia coli* JM109 colonies containing these various plasmids. In all cases, plasmid-containing *Escherichia coli* displayed yellow due to meta-cleavage, suggesting that the hybrid bphA1 genes had functioned normally. Next, *Escherichia coli* JM109 containing these various plasmids and pSHF1072 were cultured in an LB medium supplemented with 100 µg/ml ampicillin and 0.2 mM isopropyl-1-thio-β-D-galactopyranoside (IPTG) overnight at 37°C. Various substrates analogous to biphenyl were added to each culture solution at a concentration of 100 µg/ml. The presence or the absence of substrate degradation was then confirmed based on displaying of yellow. 6 types of substrates verified herein were 1-phenylnaphthalene, 2-phenylnaphthalene, 6-hydroxyflavanone, 7-hydroxyflavanone, 7-hydroxyflavone, and 5,7-hydroxyflavone. As a result, *Escherichia coli* containing the plasmid pBPA715-707BC that has hybrid bphA1 [hereinafter, bphA1 (715-707)] of the KF715 strain and the KF707 strain degraded 4 types of substrates: 1-phenylnaphthalene, 2-phenylnaphthalene, 6-hydroxyflavanone, and 7-hydroxyflavanone, showing broad spectrum of substrate specificity. Moreover, in the cases of 1-phenylnaphthalene and 2-phenylnaphthalene, such *E. coli* containing the plasmid pBPA715-707BC displayed yellow in a stronger manner than did by *Escherichia coli* having pSHF 1072. It was thus demonstrated that such *E. coli* containing the plasmid pBPA715-707BC has strong activity of degrading 1-phenylnaphthalene and 2-phenylnaphthalene.

The bphA1 (715-707) gene sequence was analyzed. This gene sequence encodes a full-length amino acid sequence (consisting of 458 amino acids) that is the same as that of the bphA1 gene derived from the *Pseudomonas pseudoalcaligenes* KF707 strain except that 3 amino acid residues (182^{nd}, 324^{th} and 325^{th} amino acid residues) are different between the two. Specifically, such amino acid residues in the gene derived from the KF707 strain were aspartic acid (182), serine (324), and valine (325); however, in BphA1 (715-707), the residues were glutamic acid (182), threonine (324), and isoleucine (325). Furthermore, the amino acid sequence of BphA1 (715-707) differed from the amino acid sequence of BphA1 (2072) at 7 positions. In addition, the amino acid sequence of BphA1 (715-707) is shown in SEQ ID NO: 2 and the nucleotide sequence of the same is shown in SEQ ID NO: 1.

*Escherichia coli* JM109 into which the plasmid pBPA715-707BC had been incorporated therein (pBPA715-707BC) was internationally deposited under the Budapest Treaty on February 2, 2004 (original deposition) under accession No. FERM BP-10210 with the International Patent Organism Depositary (Tsukuba Central 6, 1-1-1 Higashi, Tsukuba, Ibaraki, Japan), the National Institute of Advanced Industrial Science and Technology.

### [Example 3] Preparation of Escherichia coli transformant

Procedures for preparing *Escherichia coli* transformants are as described below.

The recombinant *Escherichia coli* JM109 having the modified aromatic ring dioxygenase gene, aromatic ring dihydrodiol dehydrogenase gene, and aromatic ring diol oxygenase gene prepared in Examples 1 and 2, that is, *Escherichia coli* (pSHF1072) or *Escherichia coli* (pBPA715-707BC), was subjected to liquid culture in an LB medium (1% trypton, 0.5% yeast extract, and 1% NaCl) supplemented with 150 µg/ml ampicillin (Ap) until the first half of the logarithmic phase. The resultant was suspended in glycerol to a final concentration of approximately 30% and then placed in a deep freezer at a temperature between - 70°C and - 80°C, thereby preparing a glycerol stock strain. Furthermore, as a control, *Escherichia coli* (JM109 strain) having an Ap-resistant vector alone, such as pUC118, was also similarly cultured, so as to prepare a glycerol stock strain.

### [Example 4] Coculture of Escherichia coli transformants with substrates

To initiate conversion reaction from substrates to picolinic acids through coculture with *Escherichia coli* (pSHF1072) or *Escherichia coli* (pBPA715-707BC), 1 ml of the above glycerol stock strain prepared in Example 3 was suspended in 100 ml of an LB medium (a 500 ml Erlenmeyer flask or Sakaguchi flask was used) supplemented with 150 µg/ml ampicillin (Ap) and then cultured at 120 rpm and 30°C for 6 to 7 hours (preculture). Hence, OD600 nm reached approximately 1. Next, 7 ml to 8 ml of each preculture solution was put in 100 ml of an M9 medium (a 500 ml Erlenmeyer flask or Sakaguchi flask was used) supplemented with 150 µg/ml Ap, 1 mM (final concentration) isopropyl-1-thio-β-D-galactopyranoside (IPTG), 0.4% (w/v) glucose, and 10 mg of a substrate ( Sambrook, J., Russell, D. W., "Molecular Cloning: A Laboratory Manual," Third edition, Cold Spring Harbor Laboratory Press, 2001), followed by 2 days of culture at 120 rpm and 30°C (coculture). In addition, each substrate that had been previously dissolved in a small amount of DMSO (concentration of 100 mg/ml) was added to the medium. On day 2 of culture, 100 ml of methanol was added and the resultant solution was agitated for 30 minutes. Lipid components containing the thus generated picolinic acid compounds were extracted, and then the supernatant was recovered by centrifugation at 8,000 rpm for 5 minutes.

### [Example 5] HPLC analysis of conversion products

Products obtained by reaction of *Escherichia coli* (pSHF1072) or *Escherichia coli* (pBPA715-707BC) with various substrates according to Example 4 were analyzed by HPLC. HPLC conditions employed for analysis are as described below. Detection was performed using a photodiode array detector (HITACHI L7100), so as to detect 200-nm to 500-nm absorptions.
Column: Waters Xterra MSC₁₈ (5 µm), 4.6 × 100 mm
Flow rate: 1.0 mL/min.
Solvent: A = 5% CH₃CN and 20 mM phosphoric acid and B = 95% CH₃CN and 20 mM phosphoric acid
0 → 3 minutes: A 100%
3 → 20 minutes: A 100% → B 100% (linear gradient)
20 → 30 minutes: B 100%
Temperature: room temperature

### [Example 6] Purification and identification of conversion products

An equivalent amount of methanol was added to 1 L (at a scale 10 times greater than that in Example 4) of a mixed culture solution of *Escherichia coli* (pSHF1072) or *Escherichia coli* (pBPA715-707BC) and each substrate for which conversion was confirmed in Example 5. Each resultant solution was agitated at room temperature for 2 hours. The resultant was centrifuged at 7,000 rpm for 10 minutes, thereby recovering the supernatant. The supernatant was concentrated to an amount of approximately 500 ml *in vacuo,* adjusted to pH 5, and then subjected twice to extraction with 500 ml of ethyl acetate. The ethyl acetate layer was concentrated *in vacuo,* thereby obtaining an extract containing products. The extract was subjected to thin layer chromatography (TLC) using silica gel [0.25 nm Silica Gel 60 (Merck)], so as to confirm conversion products. Subsequently, the products were subjected to column chromatography using a silica gel column [20 × 250 mm, Silica Gel 60 (Merck)] or the like, thereby obtaining pure products.

In addition, TLC development solvents used for each substrate are as described below:
Flavanone: CH₂Cl₂-MeOH-H₂O (3 : 1 : 0.1);
Flavone: CH₂Cl₂-MeOH (3 : 1);
6-hydroxyflavanone: CH₂Cl₂-MeOH-H₂O (6 : 1 : 0.1);
6-hydroxyflavone: CH₂Cl₂-MeOH-H₂O (6 : 1 : 0.1);
7-hydroxyflavanone: CH₂Cl₂-MeOH-H₂O (3 : 1 : 0.1);
2-phenylquinoline: CH₂Cl₂-MeOH-H₂O (3 : 1 : 0.1);
2-phenylbenzoxazole: CH₂Cl₂-MeOH-H₂O (5 : 1 : 0.1);
Biphenyl: CH₂Cl₂-MeOH-H₂O (4 : 1 : 0.1);
(trans-)chalcone: CH₂Cl₂-MeOH-H₂O (3 : 1 : 0.1);
3-phenyl-1-indanone: CH₂Cl₂-MeOH (4 : 1); and
2-phenylnaphthalene: CH₂Cl₂-MeOH (4 : 1).

Furthermore, development solvents for silica gel column chromatography used for each substrate are as shown below:
Flavanone: CH₂Cl₂-MeOH (4 : 1);
Flavone: CH₂Cl₂-MeOH-H₂O (4 : 1 : 0.1);
6-hydroxyflavanone: CH₂Cl₂-MeOH-H₂O (3 : 1 : 0.1);
6-hydroxyflavone: CH₂Cl₂-MeOH-H₂O (6 : 1 : 0.1);
7-hydroxyflavanone: CH₂Cl₂-MeOH-H₂O (3 : 1 : 0.1);
2-phenylquinoline: CH₂Cl₂-MeOH-H₂O (4 : 1 : 0.1);
2-phenylbenzoxazole: CH₂Cl₂-MeOH-H₂O (5 : 1 : 0.05);
Biphenyl: CH₂Cl₂-MeOH-H₂O (6 : 1 : 0.1);
(trans-)chalcone: CH₂Cl₂-MeOH-H₂O (3 : 1 : 0.1);
3-phenyl-1-indanone: CH₂Cl₂-MeOH-H₂O (4 : 1: 0.1); and
2-phenylnaphthalene: CH₂Cl₂-MeOH-H₂O (5 : 1 : 0.1).

### (1) Identification of conversion product of flavanone

An experiment of converting flavanone using *Escherichia coli* (pSHF1072) and *Escherichia coli* (pBPA715-707BC) (100 ml each) was conducted and then the resulting conversion rates were compared by HPLC. As a result, the conversion rates of these *E. coli* recombinants were found to be equivalent. Hence, an experiment (1 L) of converting flavanone using *Escherichia coli* (pSHF1072) was conducted. When the crude extract (469 mg) was subjected to TLC, generation of a product with an Rf value of 0.45 was demonstrated. This product was purified by silica gel chromatography to obtain the purified product, compound 1 (23.8 mg). The molecular ion peak (M+H)⁺ of compound 1 was observed at *m*/*z* 270 by FAB-MS (positive, matrix glycerol). Furthermore, the precise molecular weight of this ion as measured by high-resolution FAB-MS was calculated to be 270.0787. As a result, the molecular formula of this ion was found to be C₁₅H₁₁NO₄ (calculated to be 270.0767). Hence, the molecular formula of the converted compound was determined to be C₁₅H₁₁NO₄.

When compound 1 was caused to react with phenacyl bromide and TEA in CH₂Cl₂, phenacyl ester was obtained. Accordingly, the presence of carboxylic acid in compound 1 was confirmed. Next, ¹H-¹H DQF, HMQC, and HMBC of compound 1 were measured and analyzed, revealing that the flavanone A-ring and the flavanone C-ring remained intact. At this time, remaining elements composing compound 1 strongly suggested the presence of a pyridine ring in compound 1. This was also confirmed from *J*_{2',3'} = 7.7 Hz and *J*_{3',4'} = 7.6 Hz and from the ¹³C NMR chemical shift (δ_{c} 124.3, δ_{c} 124.3, δ_{c} 138.7, δ_{c} 148.8, and δ_{c} 157.3) of the carbon inferred to compose the pyridine ring. Since ¹H- ¹H vicinal spin coupling was observed in C-2'-C-3'-C-4' composing the pyridine ring, it was confirmed that carboxylic acid binds to C-5'. As a result, compound 1 was identified as 6-(4-oxo-chroman-2-yl)-pyridine-2-carboxylic acid. This was a novel compound. The structure and NMR data of this compound are as shown below and listed in Table 1. In addition, numbers used in the chemical structural formula correspond to numbers in the NMR table and do not correspond to numbers in the identified IUPAC name (the same applies to the following description).

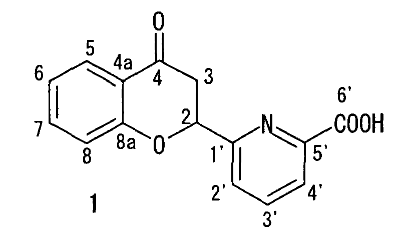

**Table 1**

| Compound 1 (6-(4-oxo-chroman-2-yl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | 5.84 (dd 2.8, 11.6) | 78.9 |
| 3 | 3.03 (dd 2.8, 17.0) | 41.6 |
| | 3.35 (dd 11.6, 17.0) | |
| 4 | | 191.1 |
| 4a | | 120.9 |
| 5 | 7.78 (dd 1.5, 7.3) | 126.4 |
| 6 | 7.11 (dd 7.0, 7.3) | 121.8 |
| 7 | 7.61 (ddd 1.5, 7.0, 8.5) | 136.4 |
| 8 | 7.15(d8.5) | 118.1 |
| 8a | | 160.5 |
| 1' | | 157.3 |
| 2' | 8.02 (d 7.7)* | 124.3 |
| 3' | 8.07 (dd 7.6, 7.7) | 138.7 |
| 4' | 7.87 (d 7.6)* | 124.3 |
| 5' | | 148.8 |
| 6' | | 166.1 |

| | | |
|---|---|---|
| * Interchangeable | | |

### (2) Identification of conversion product of flavone

An experiment of converting flavone using *Escherichia coli* (pSHF1072) (1 L) was conducted. When the thus obtained crude extract (526 mg) was subjected to TLC, generation of a product with an Rf value of 0.3 was demonstrated. This product was purified by silica gel chromatography to obtain the pure product, compound 2 (28.0 mg). The molecular ion peak (M+H)⁺ of compound 2 was observed at *m*/*z* 268 by FAB-MS. Based on ¹H and ¹³C NMR data, the molecular formula of compound 2 was determined to be C₁₅H₉NO₄.

Next, ¹H-¹H DQF COSY, HMQC, and HMBC of compound 2 were measured. The NMR spectrum of compound 2 was broad, so that the obtained structural information was insufficient. As also analyzed by comparison with compound 1, compound 2 was determined to be 6-(4-oxo-4H-chromen-2-yl)-pyridine-2-carboxylic acid. This was a novel compound. The structure and NMR data of this compound are as shown below and listed in Table 2.

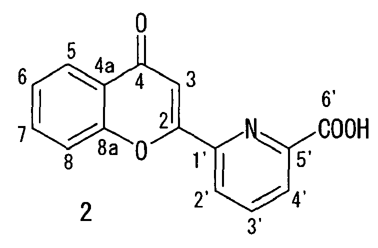

**Table 2**

| Compound 2 (6-(4-oxo-4H-chromen-2-yl)-pyridine-2-carboxylic acid) 400MHz ¹H NMR and ¹³C NMR data (in DMSO-*d*_{*6*}) | | |
|---|---|---|
| Position | δ_{H} | δ_{C} |
| 2 | | 161.3 |
| 3 | 7.33 (s) | 108.0 |
| 4 | | 177.2 |
| 4a | | 123.7 |
| 5 | 8.05 (d 7.5) | 124.8 |
| 6 | 7.51 (ddd 1.8, 7.5, 7.5) | 125.6 |
| 7 | 7.83 | 134.4 |
| 8 | 7.83 | 118.7 |
| 8a | | 155.7 |
| 1' | | 155.1 |
| 2' | 8.14 (d 7.4)* | 126.1* |
| 3' | 8.08 (dd 7.4, 7.4) | 138.2 |
| 4' | 8.23 (d 7.4)* | 122.1* |
| 5' | | 147.7 |
| 6' | | 167.8 |

| | | |
|---|---|---|
| * Interchangeable | | |

### (3) Identification of conversion product of 6-hydroxyflavanone

An experiment of converting 6-hydroxyflavanone using *Escherichia coli* (pSHF1072) (1 L) was conducted. When the thus obtained crude extract (124 mg) was subjected to TLC, generation of a product with an Rf value of 0.24 was demonstrated. This product was purified by silica gel chromatography to obtain the pure product, compound 3 (21.5 mg). The molecular ion peak (M+H)⁺ of compound 3 was observed at *m*/*z* 286 by FAB-MS. Based on ¹H and ¹³C NMR data, the molecular formula of compound 3 was determined to be C₁₅H₁₁NO₅.

Next, ¹H-¹H DQF COSY, HMQC, and HMBC of compound 3 were measured. The NMR spectrum of compound 3 was broad, so that the obtained structural information was insufficient. As also analyzed by comparison with compound 1, compound 3 was determined to be 6-(6-hydroxy-4-oxo-4H-chroman-2-yl)-pyridine-2-carboxylic acid. This was a novel compound. The NMR data of this compound are listed in Table 3.

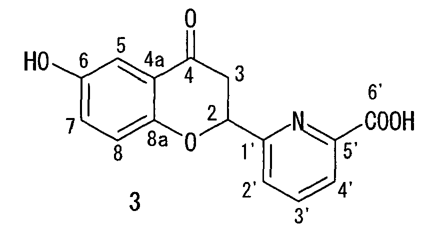

**Table 3**

| Compound 3 (6-(6-hydroxy-4-oxo-4H-chroman-2-yl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | 5.78 (d 11.6) | 79.4 |
| 3 | 2.88 (d 17.2) | 42.8 |
| | 3..10 (dd 11.6, 17.2) | |
| 4 | | 191.4 |
| 4a | | 121.0 |
| 5 | 7.15 (d 3.1) | 110.0 |
| 6 | | 152.0 |
| 7 | 7.08 (dd 3.1, 8.9) | 124.6 |
| 8 | 6.97 (d 8.9) | 119.0 |
| 8a | | 153.9 |
| 1' | | 157.2 |
| 2' | 7.73 (d 6.8)* | 122.1* |
| 3' | 7.97 (obs) | 138.3 |
| 4' | 7.97 (obs)* | 123.6* |
| 5' | | 153.7 |
| 6' | | 167.5 |

| | | |
|---|---|---|
| * Interchangeable | | |

### (4) Identification of conversion product of 6-hydroxyflavone

An experiment of converting 6-hydroxyflavone using *Escherichia coli* (pSHF1072) (1 L) was conducted. When the thus obtained crude extract (113 mg) was subjected to TLC, generation of a product with an Rf value of 0.3 was demonstrated. This product was purified by silica gel chromatography to obtain the pure product, compound 4 (37.0 mg). The molecular ion peak (M+H)⁺ of compound 4 was observed at *m*/*z* 284 by FAB-MS (positive, matrix glycerol). Based on ¹H and ¹³C NMR data, the molecular formula of compound 4 was determined to be C₁₅H₉NO₅.

Next, ¹H-¹H DQF COSY, HMQC, and HMBC of compound 4 were measured. The NMR spectrum of compound 4 was broad, so that the obtained structural information was insufficient. As also analyzed by comparison with compound 1, compound 4 was determined to be 6-(6-hydroxy-4-oxo-4H-chromen-2-yl)-pyridine-2-carboxylic acid. This was a novel compound. The NMR data of this compound are listed in Table 4.

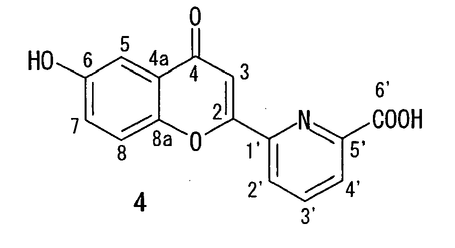

**Table 4**

| Compound 4 (6-(6-hydroxy-4-oxo-4H-chromen-2-yl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | | 160.5 |
| 3 | 7.30 (s) | 107.1 |
| 4 | | 177.0 |
| 4a | | 124.6 |
| 5 | 7.34 (d 2.6) | 107.5 |
| 6 | | 155.1 |
| 7 | 7.30 (dd 2.6, 9.2) | 123.3 |
| 8 | 7.68 (d 9.2) | 119.9 |
| 8a | | 149.2 |
| 1' | | 155.1 |
| 2' | 8.27 (br s)* | 122.6* |
| 3' | 8.14 (obs) | 138.6 |
| 4' | 8.14 (obs)* | 125.9* |
| 5' | | 148.2 |
| 6' | | 166.3 |

| | | |
|---|---|---|
| * Interchangeable | | |

### (5) Identification of conversion product of 7-hydroxyflavanone

An experiment of converting 7-hydroxyflavanone using *Escherichia coli* (pSHF1072) (1 L) was conducted. When the thus obtained crude extract (113 mg) was subjected to TLC, generation of a product with an Rf value of 0.3 was demonstrated. This product was purified by silica gel chromatography to obtain the pure product, compound 5 (68.5 mg). The molecular ion peak (M+H)⁺ of compound 5 was observed at *m*/*z* 286 by FAB-MS (positive, matrix glycerol). Based on ¹H and ¹³C NMR data, the molecular formula of compound 5 was determined to be C₁₅H₁₁NO₅.

Next, ¹H-¹H DQF COSY, HMQC, and HMBC of compound 5 were measured. The NMR spectrum of compound 5 was broad, so that the obtained structural information was insufficient. As also analyzed by comparison with compound 1, compound 5 was determined to be 6-(7-hydroxy-4-oxo-4H-chroman-2-yl)-pyridine-2-carboxylic acid. This was a novel compound. The NMR data of this compound are listed in Table 5.

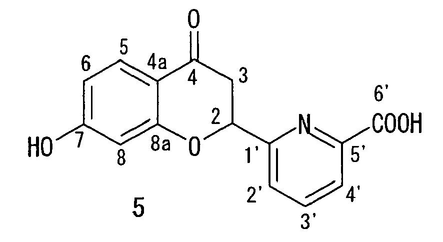

**Table 5**

| Compound 5 (6-(7-hydroxy-4-oxo-4H-chroman-2-yl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | 5.82 (br d 10.5) | 79.4 |
| 3 | 2.80 (br d 14.5) | 42.2 |
| | 3.05 (br dd 10.5, 14.5) | |
| 4 | | 189.2 |
| 4a | | 113.4 |
| 5 | 7.62 (d 8.3) | 128.4 |
| 6 | 6.53 (d 8.3) | 111.0 |
| 7 | | 165.2* |
| 8 | 6.41 (s) | 102.8 |
| 8a | | 162.6 |
| 1' | | 157.1 |
| 2' | 7.97 (br s)* | 123.7** |
| 3' | 7.97 (obs) | 138.2 |
| 4' | 7.97 (obs)* | 122.5** |
| 5' | | 153.5 |
| 6' | | 167.9 |

| | | |
|---|---|---|
| *, ** Interchangeable | | |

### (6) Identification of conversion product of 2-phenylquinoline

An experiment of converting 2-phenylquinoline using *Escherichia coli* (pSHF1072) and *Escherichia coli* (pBPA715-707BC) (100 ml each) was conducted and then the resulting conversion rates were compared by HPLC. As a result, the conversion rates of these *E. coli* recombinants were found to be equivalent. Hence, an experiment of converting 2-phenylquinoline using *Escherichia coli* (pSHF1072) (1 L) was conducted. When the crude extract (210 mg) was subjected to TLC, generation of a product with an Rf value of 0.3 was demonstrated. This product was purified by silica gel chromatography, and then purified by preparative HPLC (column: Shiseido Capsule Pack SG; diameter: 10 mm × 250 mm; solvent: 35% MeOH; and 0.2% TFA) to obtain the pure product, compound 6 (21.3 mg).

The molecular ion peak (M+H)⁺ of compound 6 was observed at *m*/*z* 251 by FAB-MS. Based on ¹H and ¹³C NMR data, the molecular formula of compound 6 was determined to be C₁₅H₁₀N₂O₂.

Next, ¹H-¹H DQF COSY, HMQC, and HMBC of compound 6 were measured. As also analyzed based on information obtained from such data and analyzed by comparison with compound 1, compound 6 was determined to be 6-quinolin-2-yl-pyridine-2-carboxylic acid. This was a novel compound. The structure and NMR data of this compound are as shown below and listed in Table 6.

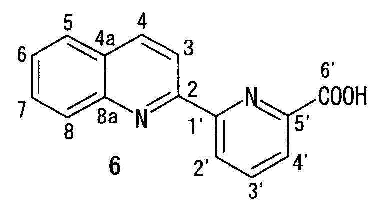

**Table 6**

| Compound 6 (6-quinolin-2-yl-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | | 154.4 |
| 3 | 8.70 (d 8.6) | 118.6 |
| 4 | 8.55 (d 8.6) | 137.2 |
| 4a | | 127.9 |
| 5 | 8.04 (d 7.9) | 127.8 |
| 6 | 7.64 (dd 7.4, 7.9) | 127.1 |
| 7 | 7.81 (dd 7.4, 8.3) | 130.0 |
| 8 | 8.11 (d 8.3) | 129.0 |
| 8a | | 146.9 |
| 1' | | 154.9 |
| 2' | 8.79 (d 6.9)* | 124.0* |
| 3' | 8.18 (dd 6.9, 7.7) | 138.6 |
| 4' | 8.15 (d 7.7)* | 125.2* |
| 5' | | 147.8 |
| 6' | | 165.7 |

| | | |
|---|---|---|
| * Interchangeable | | |

### (7) Identification of conversion product of 2-phenyl-benzoxazole

An experiment of converting 2-phenylbenzoxazole using *Escherichia coli* (pSHF1072) (1 L) was conducted. When the crude extract (74 mg) was subjected to TLC, generation of a product with an Rf value of 0.4 was demonstrated. This product was purified by silica gel chromatography, and then purified by preparative HPLC (column: Shiseido Capsule Pack SG; diameter: 10 mm × 250 mm; solvent: 50% MeOH; and 0.2% TFA) to obtain the pure product, compound 7 (18.0 mg).

The molecular ion peak (M+H)⁺ of compound 7 was observed at *m*/*z* 241 by FAB-MS. Based on ¹H and ¹³C NMR data, the molecular formula of compound 7 was determined to be C₁₃H₈N₂O₃.

Next, ¹H-¹H DQF COSY, HMQC, and HMBC of compound 7 were measured. As also analyzed based on information obtained from such data and analyzed by comparison with compound 1, compound 7 was determined to be 6-benzoxazol-2-yl-pyridine-2-carboxylic acid. This was a novel compound. The structure and NMR data of this compound are as shown below and listed in Table 7.

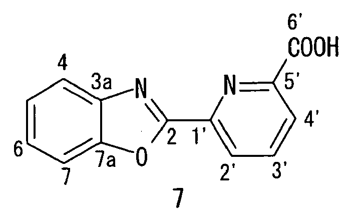

**Table 7**

| Compound 7 (6-benzoxazol-2-yl-pyridine-2-carboxylic acid) 400 MHz ¹H NMR and ¹³C NMR data (in DMSO-*d*_{*6*}) | | |
|---|---|---|
| Position | δ_{H} | δ_{C} |
| 2 | | 160.6 |
| 3a | | 141.0 |
| 4 | 7.88 (d 8.4) | 120.4 |
| 5 | 7.46 (dd 7.6, 8.4) | 125.2 |
| 6 | 7.51 (dd 7.6, 8.4) | 126.4 |
| 7 | 7.88 (dd 8.4) | 111.4 |
| 7a | | 150.5 |
| 1' | | 149.1 |
| 2' | 8.79 (d 6.9)* | 126.3* |
| 3' | 8.22 (obs) | 139.1 |
| 4' | 8.22 (obs)* | 126.7* |
| 5' | | 145.2 |
| 6' | | 165.6 |

| | | |
|---|---|---|
| * Interchangeable | | |

### (8) Identification of conversion product of biphenyl

An experiment of converting biphenyl using *Escherichia coli* (pSHF1072) and *Escherichia coli* (pBPA715-707BC) (100 ml each) was conducted and then the resulting conversion rates were compared by HPLC. As a result, the conversion rate of the latter *E. coli* recombinant (pBPA715-707BC) was found to be 1.5 times higher than that of the *E. coli* (pSHF1072) recombinant. Hence, an experiment of converting biphenyl using *Escherichia coli* (pBPA715-707BC) (1 L) was conducted. When the crude extract (121.7 mg) was subjected to TLC (CH₂Cl₂ : MeOH : H₂O = 4 : 1 : 0.1), generation of a product with an Rf value of 0.4 was demonstrated. This product was purified by silica gel chromatography (CH₂Cl₂ : MeOH : H₂O = 6 : 1 : 0.1), and then purified by preparative HPLC (column: Shiseido Capsule Pack SG; diameter: 10 mm × 250 mm; solvent: 60% MeOH; and 0.2% TFA) to obtain the pure product, compound 8 (79.2 mg).

The molecular ion peak (M+H)⁺ of compound 8 was observed at *m*/*z* 200 by FAB-MS. Based on ¹H and ¹³C NMR data, the molecular formula of compound 8 was determined to be C₁₂H₉NO₂.

Next, ¹H-¹H DQF COSY, HMQC, and HMBC of compound 8 were measured. As also analyzed based on information obtained from such data and as analyzed by comparison with compound 1, compound 8 was determined to be 6-phenyl-pyridine-2-carboxylic acid. The structure and NMR data of this compound are as shown below and listed in Table 8.

This compound is a known substance (JP Patent Publication (Kokai) No. 55-000307 A (1980)) as registered in CAS. However, a technique for preparing such compound through a bioconversion reaction using a recombinant microorganism having a necessary converting enzyme gene cluster is a novel technique. Moreover, converting enzymes or genes encoding such enzymes required for preparing compound 8 have been revealed for the first time by the present invention.

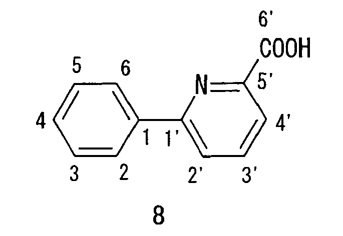

**Table 8**

| Compound 8 (6-phenyl-pyridine-2-carboxylic acid) 400 MHz ¹H NMR and ¹³C NMR data (in DMSO-*d*_{*6*}) | | |
|---|---|---|
| Position | δ_{H} | δ_{C} |
| 1 | | 137.6 |
| 2 | 8.17 (d 7.2) | 126.7 |
| 3 | 7.52 (dd 7.2, 7.2) | 128.7 |
| 4 | 7.48 (dd 7.2, 7.2) | 129.4 |
| 5 | 7.52 (dd 7.2, 7.2) | 128.7 |
| 6 | 8.17 (d 7.2) | 126.7 |
| 1' | | 155.8 |
| 2' | 7.98 (d 7.7)* | 123.1* |
| 3' | 8.05 (dd 7.6, 7.7) | 138.4 |
| 4' | 8.19 (d 7.6)* | 123.2* |
| 5' | | 148.1 |
| 6' | | 166.0 |

| | | |
|---|---|---|
| *Interchangeable | | |

### (9) Identification of conversion product of (trans-)chalcone

An experiment of converting (trans-)chalcone using *Escherichia coli* (pSHF1072) or *Escherichia coli* (pBPA715-707BC) (1 L) was conducted. When the crude extract (456 mg) was subjected to TLC, generation of a product with an Rf value of 0.35 was demonstrated. This product was purified by silica gel chromatography to obtain the pure product, compound 9 (30.7 mg).

The molecular ion peak (M+H)⁺ of compound 9 was observed at *m*/*z* 256 by FAB-MS. Based on ¹H and ¹³C NMR data, the molecular formula of compound 9 was determined to be C₁₅H₁₃NO₃.

Next, ¹H-¹H DQF COSY, HMQC, and HMBC of compound 9 were measured. The NMR spectrum of compound 9 was broad, so that the obtained structural information was insufficient. As also analyzed by comparison with compound 1, compound 9 was determined to be 6-(3-oxo-3-phenyl-propyl)-pyridine-2-carboxylic acid. This was a novel compound. The structure and NMR data of this compound are as shown below and listed in Table 9.

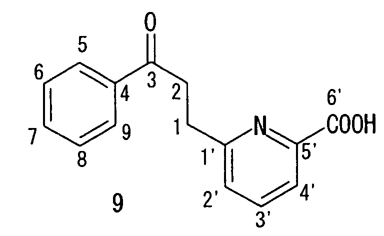

**Table 9**

| Compound 9 (6-(3-oxo-3-phenyl-propyl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 1 | 3.59 (br s)* | 31.4* |
| 2 | 3.67 (br s)* | 39.8 |
| 3 | | 200.0 |
| 4 | | 137.3 |
| 5 | 8.03 (d 6.3) | 129.2 |
| 6 | 7.51 (dd 6.3, 7.2) | 129.7 |
| 7 | 7.63 (dd 7.2, 7.2) | 134.6 |
| 8 | 7.51 (dd 6.3, 7.2) | 129.7 |
| 9 | 8.03 (d 6.3) | 129.2 |
| 1' | | 158.8 |
| 2' | 7.51 (br s)** | 129.2** |
| 3' | 8.41 (obs) | 137.3 |
| 4' | 8.41 (obs)** | 125.5** |
| 5' | | 146.1 |
| 6' | | 169.1 |

| | | |
|---|---|---|
| *, ** Interchangeable | | |

### (10) Identification of conversion product of 3-phenyl-1-indanone

An experiment of converting 3-phenyl-1-indanone using *Escherichia coli* (pSHF1072) (1 L) was conducted. When the crude extract (79 mg) was subjected to TLC, generation of a product with an Rf value of 0.2 was demonstrated. This product was purified by silica gel chromatography, and then purified by preparative HPLC (column: Shiseido Capsule Pack SG; diameter: 10 mm × 250 mm; solvent: 50% MeOH; and 0.2% TFA) to obtain the pure product, compound 10 (10.0 mg).

The molecular ion peak (M+H)⁺ of compound 10 was observed at *m*/*z* 253 by FAB-MS. Based on ¹H and ¹³C NMR data, the molecular formula of compound 10 was determined to be C₁₅H₁₁NO₃.

Next, ¹H-¹H DQF COS, HMQC, and HMBC of compound 10 were measured. As also analyzed based on information obtained from such data and analyzed by comparison with compound 1, compound 10 was determined to be 6-(3-oxo-indan-1-yl)-pyridine-2-carboxylic acid. This was a novel compound. The structure and NMR data of this compound are as shown below and listed in Table 10.

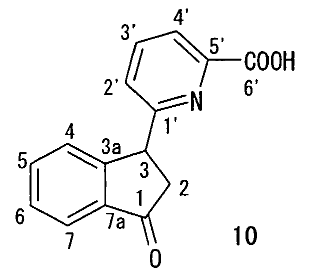

**Table 10**

| Compound 10 (6-(3-oxo-indan-1-yl)-pyridine-2-carboxylic acid) 400 MHz ¹H NMR and ¹³C NMR data (in DMSO-*d*_{*6*}) | | |
|---|---|---|
| Position | δ_{H} | δ_{C} |
| 1 | | 205.1 |
| 2 | 3.07 (dd 3.7, 17.9) | 42.9 |
| | 3.13 (dd 5.4, 17.9) | |
| 3 | 4.93 (dd 3.7, 5.4) | 45.3 |
| 3a | | 156.5 |
| 4 | 7.34 (d 7.5) | 126.5 |
| 5 | 7.61 (dd 7.5, 7.5) | 135.0 |
| 6 | 7.45 (dd 7.5, 7.5) | 128.0 |
| 7 | 7.69 (d 7.5) | 122.9 |
| 7a | | 136.0 |
| 1' | | 161.6 |
| 2' | 7.91 (d 6.8)* | 123.1* |
| 3' | 7.96 (dd 6.8, 7.5) | 138.5 |
| 4' | 7.69 (d 7.5)* | 126.1 * |
| 5' | | 148.2 |
| 6' | | 165.9 |

| | | |
|---|---|---|
| *Interchangeable | | |

### (11) Identification of conversion product of 2-phenylnaphthalene

An experiment of converting 2-phenylnaphthalene using *Escherichia coli* (pSHF1072) and *Escherichia coli* (pBPA715-707BC) (100 ml each) was conducted and then the resulting conversion rates were compared by HPLC. As a result, the conversion rate of the latter *E. coli* recombinant (pBPA715-707BC) was found to be 1.5 times higher than that of the *E. coli* (pSHF1072) recombinant. Hence, an experiment of converting 2-phenylnaphthalene using *Escherichia coli* (pBPA715-707BC) (1 L) was conducted. When the crude extract (101 mg) was subjected to TLC, generation of a product with an Rf value of 0.2 was demonstrated. This product was purified by silica gel chromatography, and then purified by preparative HPLC (column: Shiseido Capsule Pack SG; diameter: 10 mm × 250 mm; solvent: 70% MeOH; and 0.2% TFA) to obtain the pure product, compound 11 (20.1 mg).

The molecular ion peak (M+H)⁺ of compound 11 was observed at *m*/*z* 250 by FAB-MS. Based on ¹H and ¹³C NMR data, the molecular formula of compound 11 was determined to be C₁₆H₁₁NO₂.

Next, ¹H-¹H DQF COS, HMQC, and HMBC of compound 11 were measured. As also analyzed based on information obtained from such data and analyzed by comparison with compound 1, compound 11 was determined to be 6-naphthalene-2-yl-pyridine-2-carboxylic acid. This was a novel compound. The structure and NMR data of this compound are as shown below and listed in Table 11.

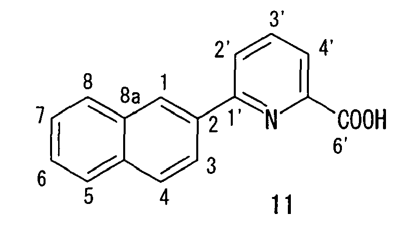

**Table 11**

| Compound 11 (6-naphthalene-2-yl-pyridine-2-carboxylic acid) 400 MHz ¹H NMR and ¹³C NMR data (in DMSO-*d*_{*6*}) | | |
|---|---|---|
| Position | δ_{H} | δ_{C} |
| 1 | 8.75 (s) | 126.3 |
| 2 | | 133.0* |
| 3 | 8.35*** | 124.4 |
| 4 | 8.05*** | 128.3 |
| 4a | | 133.4 |
| 5 | 7.97 (m) | 127.6 |
| 6 | 7.57*** | 126.9 |
| 7 | 7.57*** | 126.6 |
| 8 | 8.05*** | 128.7 |
| 8a | | 135.1* |
| 1' | | 155.8 |
| 2' | 8.37 (d 7.3)** | 123.7** |
| 3' | 8.11 (dd 7.3, 7.8)** | 138.7 |
| 4' | 8.02 (d 7.8) | 123.3** |
| 5' | | 148.3 |
| 6' | | 166.2 |

| | | |
|---|---|---|
| *, **Interchangeable *** J value unknown because of overlapping signals | | |

All publications, patents, and patent applications cited herein are incorporated herein by reference in their entirety.

### Industrial Applicability

According to the present invention, a novel method for producing a picolinic acid compound is provided, wherein the picolinic acid compound is a low-molecular-weight organic compound and is produced with difficulty by organic chemical synthesis. In the method according to the present invention, a low-molecular-weight organic compound having a phenyl group (benzene ring) within a molecule as a substrate for enzyme reaction may be used. Hence, raw materials for industrial products having high added value, such as pharmaceutical preparations or agricultural chemicals, can be produced from an abundant variety of inexpensive commercial chemosynthetic substrates. Furthermore, a method for converting a phenyl group (benzene ring) into picolinic acid is provided according to the present invention. The method is useful in terms of provision of a technique for an organic chemical synthesis method.

### Sequence Listing Free Text

SEQ ID NO: 1: Synthetic gene
SEQ ID NO: 2: Synthetic protein
SEQ ID NOS: 3 and 4: Synthetic oligonucleotides
SEQ ID NOS: 5 and 6: Synthetic peptides
SEQ ID NOS: 7 and 8: Synthetic oligonucleotides (N denotes A, C, G, or T)
SEQ ID NOS: 9 and 10: Synthetic oligonucleotides

## Claims

1. A method for producing a picolinic acid compound, which comprises
reacting an aromatic compound that contains a phenyl group represented by the following formula (I), (II), or (III): 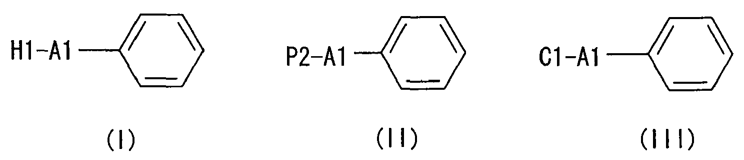 wherein, H1 is an optionally substituted heterocyclic group, A1 is a single bond or an optionally substituted C₁₋₄ alkylene group or alkenylene group, P2 is an optionally substituted phenyl group, and C1 is an optionally substituted cyclic hydrocarbon group (excluding a phenyl group), and where formula II does not represent diphenylacetylene
with aromatic ring dioxygenase, aromatic ring dihydrodiol dehydrogenase, and aromatic ring diol dioxygenase, and
obtaining a picolinic acid compound (I'), (II'), or (III'): 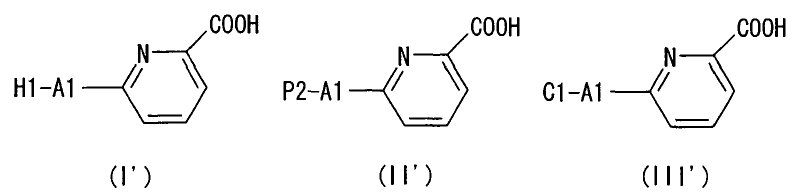 wherein H1, A1, P2, and C1 are as defined above.

2. The method according to claim 1, wherein aromatic ring dioxygenase, aromatic ring dihydrodiol dehydrogenase, and aromatic ring diol dioxygenase are those derived from biphenyl-degrading bacteria or variants thereofor modified products obtained therefrom by molecular evolution engineering techniques.

3. A method for producing a picolinic acid compound, which comprises
culturing a recombinant microorganism into which genes encoding aromatic ring dioxygenase, aromatic ring dihydrodiol dehydrogenase, and aromatic ring diol dioxygenase have been introduced in a medium containing a compound represented by the following formula (I), (II), or (III): 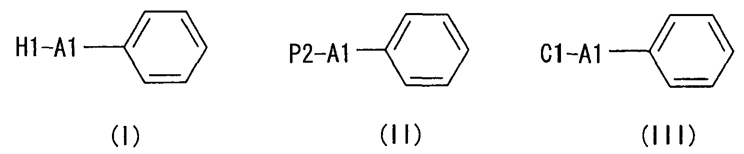 wherein H1 is an optionally substituted heterocyclic group, A1 is a single bond or an optionally substituted C₁₋₄ alkylene group or alkenylene group, P2 is an optionally substituted phenyl group, and C1 is an optionally substituted cyclic hydrocarbon group (excluding a phenyl group), and where the formula II does not represent diphenylacetylene
and
obtaining from the culture product or the microorganism a picolinic acid compound (I'), (II') or (III'): 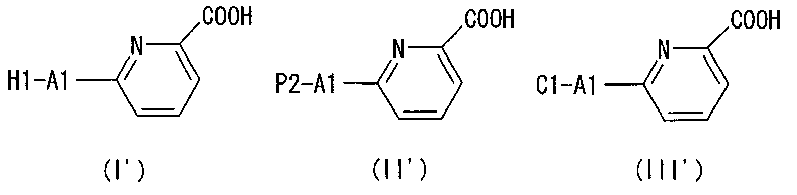 wherein H1, A1, P2, and C1 are as defined above.

4. The method according to claim 3, wherein the genes encoding aromatic ring dioxygenase, aromatic ring dihydrodiol dehydrogenase, and aromatic ring diol dioxygenase are those derived from biphenyl-degrading bacteria or variants thereof or modified products obtained therefrom by molecular evolution engineering techniques.

5. The method according to claim 3 or 4, wherein the recombinant microorganism is recombinant *Escherichia coli.*

6. The method according to any one of claims 1 to 5, wherein the compound represented by the formula (I), (II), or (III) is selected from the group consisting of flavanone, flavone, 6-hydroxyflavanone, 6-hydroxyflavone, 7-hydroxyflavanone, 2-phenylquinoline, 2-phenylbenzoxazole, biphenyl, (trans-)chalcone, 3-phenyl-1-indanone, and 2-phenylnaphthalene.

7. The method according to any one of claims 1 to 6, wherein a large subunit of aromatic ring dioxygenase is the following protein (a) or (b):
(a) a protein comprising the amino acid sequence shown in SEQ ID NO: 2; or
(b) a protein comprising a sequence derived from the amino acid sequence shown in SEQ ID NO: 2 by deletion, substitution, or addition of 1 or several amino acids and having functions of the large subunit of aromatic ring dioxygenase.

8. A protein (a) or (b) shown below:
(a) a protein comprising the amino acid sequence shown in SEQ ID NO: 2; or
(b) a protein comprising a sequence derived from the amino acid sequence shown in SEQ ID NO: 2 by deletion, substitution, or addition of 1 or several amino acids and having functions of a large subunit of aromatic ring dioxygenase.

9. A gene encoding the following protein (a) or (b):
(a) a protein comprising the amino acid sequence shown in SEQ ID NO: 2; or
(b) a protein comprising a sequence derived from the amino acid sequence shown in SEQ ID NO: 2 by deletion, substitution, or addition of 1 or several amino acids and having functions of a large subunit of aromatic ring dioxygenase.

10. A gene comprising DNA consisting of the nucleotide sequence shown in SEQ ID NO: 1.
